(19) Europäisches Patentamt
European Patent Office
Office européen des brevets

(11) EP 2 148 896 B1

(12) **EUROPEAN PATENT SPECIFICATION**

(45) Date of publication and mention of the grant of the patent:
**14.11.2012 Bulletin 2012/46**

(21) Application number: **08758584.0**

(22) Date of filing: **16.05.2008**

(51) Int Cl.:
***A61L 9/012*** *(2006.01)* ***A61L 9/04*** *(2006.01)*
***C08G 18/28*** *(2006.01)* ***C08G 18/32*** *(2006.01)*
***C08G 18/66*** *(2006.01)* ***C08G 18/42*** *(2006.01)*
***C08G 18/36*** *(2006.01)*

(86) International application number:
**PCT/EP2008/003957**

(87) International publication number:
**WO 2008/141777 (27.11.2008 Gazette 2008/48)**

(54) **COMPOSITION AND METHOD**

ZUSAMMENSETZUNG UND VERFAHREN

COMPOSITION ET PROCÉDÉ

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **22.05.2007 GB 0709781**

(43) Date of publication of application:
**03.02.2010 Bulletin 2010/05**

(73) Proprietor: **Croda International PLC**
**Goole, East Yorkshire DN14 9AA (GB)**

(72) Inventors:
- **TOLLINGTON, Peter, James**
**NL-3062 XN Rotterdam (NL)**
- **TRIET, Remo, Benjamin, Van**
**NL-2811 WC Reeuwijk (NL)**

(74) Representative: **Humphries, Martyn et al**
**Croda Europe Limited**
**Intellectual Property**
**Cowick Hall**
**Snaith**
**Goole**
**East Yorkshire DN14 9AA (GB)**

(56) References cited:
**WO-A-2004/081056 US-A- 4 853 430**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 148 896 B1

## Description

The present invention relates to structuring polymers comprising urethane and/or urea linkages and their use in the manufacture of compositions comprising a structured organic phase. The structured compositions comprise a fragrance composition and are typically suitable for use in domestic applications.

Structured, e.g. gelled, compositions for use in fragrance applications such as air fresheners or scented candles are well known. However the properties of known structuring polymers for use in fragrance applications are generally unsatisfactory. In particular, the range of fragrances which can be incorporated into known gels is generally limited by the chemical constituents of the fragrance ingredient, which can otherwise weaken or destroy the action of the structuring polymer.

Alternatively or additionally, it can be difficult to obtain a substantially transparent gel for a range of fragrances using structuring polymers known in the prior art. In particular, gellants are not available which can gel both polar and non-polar solvents, especially where translucence or clarity is desired.

Additionally, structuring polymers known in the prior art often require high temperatures for their effective incorporation into the structured compositions, these high temperatures can damage sensitive ingredients, such as fragrance ingredients, and necessitate extra safety precautions relating to the potential combustion of volatile materials at high temperatures. In-situ polymerisation of fragrance ingredients is known from the prior art, which may lower temperatures, but involves handling toxic and/or chemically reactive ingredients, and suffers from the same limitation on range of fragrances used due to the chemical interactions as described above.

Document WO2004/081056 is directed to controlled release polymeric gels. The example 3 in tables 5 and 8 shows the preparation of one of those gels: 3% by weight of a fatty acid dimer diol (Pripol 2033) and a carbodiimide functionalized polyisocyanate are mixed with 75% by weight of a lavender fragrance. The reaction between the fatty acid dimer diol and the carbodiimide functionalized polyisocyanate lead to urethane linkages. The structured composition may be used as a deodorant controlled release gel.

Gelled compositions are of particular commercial interest due, at least in part, to their attractive appearance and tactile properties. Other structured compositions, such as waxy and viscous liquid compositions, are also of commercial importance. There is a need for improved structuring polymers which can be used to make gels and other structured compositions in low polarity liquids, especially those which contain a fragrance composition. The need is particularly acute in respect of gels, but is also significant in other areas.

Desirable properties of gelled compositions depend on the exact application, but typically include; retention of shape or form, stability of the physical gel structure, uniform fragrance release over the lifetime of the article, and pleasant tactile qualities. These are desirable over a range of ambient conditions.

Structuring polymers for use in preparing gelled compositions desirably;

- produce a transparent gel with a wide range of different fragrance types (polarity, chemistry);
- produce a hard gel with acceptable tactile properties, i.e. not greasy, oily or tacky;
- produce a gel which does not creep or flow over time, for example when applied on a vertical surface such as a glass plate;
- produce a gel which is stable and shows no syneresis or bleeding due to poor compatibility or shrinkage of the polymer;
- produce a gel which retains the abovementioned physical properties over a wide range of temperature and humidity conditions; and/or
- allow production of a gelled composition at temperatures which minimise damage to the sensitive fragrance, perfume and other ingredients.

According to one aspect of the present invention there is provided a structuring polymer suitable for use for structuring an organic phase comprising a fragrance composition, wherein the structuring polymer contains fatty acid dimer/trimer containing moieties and comprises urea and/or urethane linkages, and the structuring polymer has a melting point of from 40 to 180 °C, and/or a softening point of from 30 to 160 °C, and/or the urethane and urea linkages are in a mole ratio of 7:3 or greater.

By organic phase it is meant a liquid medium which is comprised substantially or entirely of organic material. In the majority of cases the organic phase will be an oil. The term oil is used in a broad sense and comprises organic liquids, generally with a high carbon content, which may be non-polar or polar, and which are typically liquid at ambient temperature (20 °C) and atmospheric pressure (760 mmHg).

The term "structuring" or "structured" refers to the formation of a network of polymer molecules within the organic phase. The polymer chains typically link through weak intermolecular interactions to form a reversible network which imparts solid-like properties to the structured composition. The structuring can range from a slight increase in viscosity of the organic phase, to an elastic (gel) or waxy solid in which the oil is essentially fully bound within the polymer structure.

Fatty acid dimer or trimers (often referred to simply as "dimer acids" or "trimer acids") are well known polymerisation products, typically derived from unsaturated fatty acids. Principally in industry, compositions rich in oleic, linoleic and linolenic acids and mixtures thereof are used as feedstocks. The fatty acids are typically polymerised at elevated temperatures using clay catalysts.

A fatty acid dimer or trimer generally has an average molecular weight corresponding to approximately two or three molecules of the starting fatty acid respectively; dimerised oleic acid therefore has an average molecular weight corresponding to a nominally $C_{36}$ diacid. As they are manufactured, dimer/trimer fatty acids are generally unsaturated, typically corresponding to 1 or 2 ethylenic double bonds per molecule, but these double bonds may be hydrogenated in making the fatty acid dimer/trimer containing moieties of this invention.

Fatty acid dimer/trimer containing moieties suitable for use in the present invention are derived from fatty acid dimers and/or trimers.

The fatty acid dimer/trimer containing moieties may be diol derivatives (dimer diols/trimer triols) or diamine derivatives (dimer diamines/trimer triamines) of fatty acid dimer or trimers. Dimer/trimer diols are the dihydroxy alcohols which may be obtained by reducing or hydrogenating a fatty acid dimer/trimer derivative, usually the methyl ester, to the dimer/trimer diol or by dimerisation or trimerisation of a corresponding unsaturated alcohol. Dimer/trimer amines are commercially made by nitrilation of the dimer/trimer fatty acid, e.g. with ammonia, followed by hydrogenation. The trimer derivatives may, of course include three hydroxyl groups or amine groups (triol or triamine). Such triols or triamines would have a valency of three, compared to two in a fatty acid diol or diamine.

In more detail, fatty acid dimers and trimers are commercially made as distillation fractions from the polymerisation reaction described above, and typically include small proportions of monocarboxylic materials (i.e. non-dimerised or non-trimerised reagents). The proportion of such monofunctional material is desirably kept relatively low, or reduced to a negligible level, as such compounds will tend to act as chain stoppers when incorporated into the polymers. Generally the proportion of residues of such monofunctional hydroxyl or amine compounds in the material used to make the polymer will not be more than about 6 % w/w, more usually not more than about 3 wt%, and desirably not more than about 1 % w/w, of the total fatty acid dimer/trimer containing moieties used. Amounts from 0.5 to 3 % w/w, more usually 1 to 2 % w/w, of the total fatty acid dimer/trimer containing moieties used are typical, though may be reduced if required.

Trivalent trimer fatty acid dimer/trimer containing moieties may give rise to branched polymers when they are incorporated into the polymer. The proportion of such trivalent fatty acid dimer/trimer containing moieties in the material used to make the structuring polymers of the invention will not generally be more than about 80 % w/w, more usually not more than about 25 % w/w, and desirably not more than about 3 % w/w, of the total fatty acid dimer/trimer containing moieties residues. Amounts from 0 to 2 % w/w, of the total fatty acid trimer/dimer containing moiety used are typical.

It is entirely possible that the fatty acids dimer/trimer containing moieties could comprise an amine group and a hydroxyl group, thus providing a hetero-divalent (or hetero-trivalent) derivative. This is generally less preferable as it is more complex to manufacture such a moiety.

Other fatty acid dimer/trimer containing moieties suitable for use in the present invention include polymeric moieties which include dimer/trimer acid derivatives, i.e. the moiety comprises a polymer in which the fatty acid dimer/trimer (or its amine/alcohol derivative) is polymerised, optionally in conjunction with other monomers or moieties. Such polymeric moieties may conveniently be formed via polymerisation of a fatty acid dimer/trimer, or its alcohol or amine derivative, with a corresponding polyfunctional acid, alcohol, amine or isocyanate. The polyfunctional acid, alcohol, amine, or isocyanate may be of essentially any size, but in many embodiments a relatively small molecule may be preferred, for example a molecule containing from 2 to 10, especially from 2 to 6 carbon atoms.

In one embodiment such a polymeric moiety may comprise a polyester of a dimer/trimer fatty acid and a diol (e.g. Priplast™, polyester polyols, ex. Croda). Typically such a polyester comprises a small diol, e.g. containing 15 or fewer carbon atoms, though larger diols such as polypropylene glycol, polyethylene glycol or other large polyfunctional alcohols may also be used. The polymeric moiety may comprise between 2 and 10 fatty acid dimers/trimers, more preferably between 2 and 5 fatty acid dimers/trimers. The polymeric polyester moiety typically comprises hydroxyl groups at both ends, thus in effect the polyester is a polymeric diol. Similar polymeric amines can also be manufactured and used.

In an alternative embodiment fatty acid dimers/trimers and their alcohol derivatives may be copolymerised to form polymeric fatty acid dimer/trimer containing moieties.

Suitable dimer/trimer diols for use as the fatty acid dimer/trimer containing moiety in the present invention have the structure HO-($D^a$)-OH, wherein ($D^a$) represents the residue of a diol which is, or includes, a fatty acid dimer/trimer residue.

Suitable dimer/trimer diamines for use as the fatty acid dimer/trimer containing moiety in the present invention have the structure $H_2N$-($D^b$)-$NH_2$, wherein ($D^b$) represents the residue of a diamine which is, or includes, fatty acid dimer/trimer residue.

Suitable materials for use as the fatty acid dimer/trimer containing moiety include, for example the commercial product sold under the tradename Pripol™ 2033 - ex. Croda (~97% w/w dimer diol).

Suitable materials for use as the fatty acid dimer/trimer containing moiety can also be formed by the reaction

of dimer/trimer acid, such as the commercial product sold under the tradename Pripol™ 1017 - ex. Croda (~80% w/w dimer acid, ~20% w/w trimer acid), with glycols, polyols and other hydroxy-functional materials, including those described below as polymer modifying moieties of the structuring polymer. Commercial examples of polyester polymeric fatty acid dimer/trimer containing moieties include the Priplast™ range sold by Croda.

Suitable materials for use as the fatty acid dimer/trimer containing moiety can also be formed by the reaction of dimer/trimer acid, such as the commercial product sold under the tradename Pripol™ 1017 - ex. Croda (~80% w/w dimer acid, ~20% w/w trimer acid), with (poly)amines and other amine-containing materials, including those described below as polymer modifying moieties of the structuring polymer.

As mentioned above, when dimer or trimer fatty acids are manufactured, they typically contain a mixture of both dimer and trimer fatty acids. It is possible to control the level of dimer and trimer present, or to purify the dimer or trimer, and thus different levels of purity of the dimer or trimer can be obtained. However, for most applications of these products it is not necessary and, as such, uneconomical to produce highly pure dimer or trimer products. Accordingly, it should be understood that most commercially available dimer fatty acid products will contain some trimer fatty acid, typically from 95/5 (dimer/trimer) in a relatively pure dimer product, to 80/20 in a less pure form. Likewise, commercially available trimer fatty acid products will contain some dimer fatty acid, typically 50/50 (trimer/dimer). For the purpose of the present invention this is not generally of concern, as both dimer and trimer fatty acids, or their derivatives, are compatible with the present invention. However, for some embodiments it may be desirable to use predominantly, or essentially entirely either the dimer or the trimer, and this can be achieved via conventional purification techniques.

It should be noted that the term "polymer" as used herein is used irrespective of the number of repeat units or molecular weight of the materials concerned, e.g. it may cover oligomers.

The structuring polymers of the present invention contain urethane and/or urea linkages. Urethane linkages are typically obtained by reacting an isocyanate group with a hydroxyl group. Urea linkages are typically obtained by the reaction of an isocyanate group with an amine group.

Accordingly, the structuring polymer of the present invention may suitably contain monomers which are isocyanate molecules, more preferably diisocynanate molecules. Isocyanate molecules may react with the fatty acid trimer/dimer containing moiety, and where the reaction is between an isocyanate and a hydroxyl group of the fatty acid derivative a urethane linkage will be formed, and where the reaction is between an isocyanate and an amine, a urea linkage will be formed.

Generally the diisocyanate is of the formula OCN-R-NCO, where R is essentially any suitable radical. Suitable isocyanates include aromatic isocyanates, particularly diisocyanates e.g. phenyl diisocyanate, methylene bis-(4,4')-phenyl isocyanate (also known as diphenylmethane-4,4'-diisocyanate or MDI), toluene diisocyanate (TDI), tetramethylxylene diisocyanate or derivatives and variants of such materials for example modified MDI; but more usually non-aromatic diisoycanates such as alicyclic isocyanates, particularly diisocyanates e.g. methylene bis-(4,4')-cyclohexyl isocyanate (4,4'-dicyclohexylmethane diisocyanate), or isophorone diisocyanate; dimer diisocyanate; or, and particularly, alkylene isocyanates, particularly diisocyanates, more particularly $C_2$ to $C_{12}$, especially $C_2$ to $C_8$, and desirably $C_2$ to $C_6$ alkylene diisocyanates, such as 2,2,4-trimethyl-1,6-hexamethylene diisocyanate; and desirably diisocyanates of the formula: $OCN-(CH_2)_p-NCO$ where p is from 2 to 12, more particularly from 2 to 8, and especially from 2 to 6 e.g. 1,12-dodecane diisocyanate, or 1,6 hexamethylene isocyanate.

In preferred embodiments the isocyanate is one or more of:

- hexamethylene diisocyanate (HDI) (in particular HDI sold under the trademark Desmodur H),
- an HDI trimer (in particular the trimer sold under the trademark Desmodur N3600),
- dicyclohexanemethane-4,4'-diisocyanate (in particular that sold under the trademark Desmodur W), and/or
- modified MDI (in particular that sold under the trademark Desmodur CD).

The structuring polymer may contain one or more polymer modifying moieties. These are typically polyvalent alcohols or amines, typically divalent molecules, i.e. diols or diamines. These polymer modifying moieties essentially replace the fatty acid dimer/trimer containing moiety at certain points in the polymer. The polymer modifying moieties may be included to alter the properties of the polymer, e.g. to vary the gel strength, increase the solubility of the polymer, or improve the thermal stability, i.e. to increase the temperature at which the gel softens or melts. They may also provide suitable points for branching or cross-linking of the polymer.

Suitable diols for use as polymer modifying moieties include alkane diols, e.g. 2 ethylhexane-1,3 diol, αω-alkane diols such as ethylene glycol, 1,3-propane diol and 1,4-butane diol, neopentyl glycol (2,2-dimethylpropane-1,3-diol), 1,6-hexane diol and 1,10-decane diol, polyalkylene glycols particularly those made using ethylene, propylene or butylene oxide, predominantly hydroxyl ended polyester polyol polymers of dicarboxylic acids, such as adipic, azelaic, sebacic and dimer acids and their mixtures, and diols, such as those set out above (including dimer diols), partial fatty esters of polyols in which polyols such as glycerol, trimethylolpropane, sorbitol sorbitan, polyglycerol, pentaerithrytol and their alkoxylated versions, are esterified with fatty acids to give an average hydroxyl functionality close to 2, or such that two

hydroxyl groups on the ester are substantially more reactive and fatty acid esters in which the fatty acids contributes hydroxyl functionality, such as glycol and polyol esters of ricinoleic acid, 12-hydroxystearic acid and 9,10-dihydroxystearic acid. Glycerol triricinoleate (or castor oil) may suitably be used. Diols from alkoxylation of ammonia, such as diethanolamine, or hydrocarbyl, particularly alkyl, especially fatty alkyl, amines such as laurylamine and diol derivatives of epoxidised oils and fats may also be used.

Using polymeric diols as polymer modifying moieties it is possible to control the molecular weight and relative hydrophobicity of the polymer modifying moiety so it can be chosen to be similar or different to the fatty acid dimer/trimer containing moiety. This may enable more subtle adjustment of the structuring effect of the polymer on the organic phase.

When used, diol polymer modifying moieties will generally be from 0.1 to 75 % w/w, more usually from 0.1 to 50 % w/w, and desirably from 0.1 to 20 % w/w, of the total diol moieties used (i.e. combined total of diol polymer modifying moieties and diol fatty acid dimer/trimer containing moieties). Correspondingly, the proportion of diol fatty acid dimer/trimer containing moieties used will generally be from 25 to 99.9 % w/w, more usually from 50 to 99.9 %, w/w and desirably from 80 to 99.9 % w/w, of the total diol moieties used.

Amines that can be used as polymer modifying moieties, i.e. which can substitute for amine fatty acid dimer/trimer containing moieties at certain points in the polymer, include hydrocarbyl diamines particularly alkylene diamines such as ethylenediamine, 1,2- and 1,3-diaminopropane, 1,4-diaminobutane, 1,2-diamino-2-methylpropane, 1,3- and 1,5-diaminopentane, 2,2-dimethyl-1,3-propanediamine, 1,6-hexane-diamine (hexamethylenediamine), 2-methyl-1,5-pentanediamine, 1,7-diaminoheptane, 1,8-diamino-octane, 2,5-dimethyl-2,5-hexanediamine, 1,9-diaminononane, 1,10-diaminodecane and 1,12-diaminododecane, cyclic hydrocarbyl amines such as 4,4'-methylenebis(cyclohexylamine), 1,3-cyclohexanebis(methylamine), adamantane diamine and 1,8-diamino-p-menthane, aromatic diamines such as 1,2-, 1,3- and/or 1,4-phenylene diamine, 2,4,6-trimethyl-1,3-phenylenediamine, 2,3,5,6-tetramethyl-1,4-phenylenediamine, xylene and naphthalene diamine (all isomers), diaminophenanthrene (all isomers, including 9,10), 2,7-diaminofluorene, diaminonaphthalene (all isomers, including 1,5; 1,8; and 2,3) and cyclic amines such as 4-amino-2,2,6,6-tetramethylpiperidine. Such diamines may include hetero- e.g. oxygen, atoms particularly in alkyleneoxy residues. Examples of such materials include the so-called Jeffamine diamines (poly(alkyleneoxy)-diamines from Huntsman). The diamines may include further nitrogen atoms as in polyalkylene amines, which are typically of the formula: $NH_2-(CH_2CH_2NH)_mCH_2CH_2-NH_2$, where m is from 1 to about 5 and examples include diethylenetriamine and triethylenetetramine. The further nitrogen atoms may also be present as tertiary nitrogen atoms in particular as hetero-atoms in a cyclic group as in bis(aminoethyl)-N,N'-piperazine and bis(aminopropyl)-N,N'-piperazine. Such diamines may have one primary amine group and one secondary amine group as in N-ethylethylenediamine or 1-(2-aminoethyl)piperazine.

Generally when such diamine polymer modifying moieties are included the amounts will be relatively small as they may react to give (bis)-urea linkages that will lead to stiffer chains and the polymers will usually have higher melting temperatures. If included, such diamine polymer modifying moieties will generally be from 1 to 20 % w/w, more usually from 1 to 15 % w/w, and desirably from 1 to 10 % w/w, of the total diamine moieties used (i.e. combined total of diamine polymer modifying moieties and diamine fatty acid dimer/trimer containing moieties). Correspondingly, the proportion of dimer diamine residues used will generally be from 70 to 100 % w/w, more usually from 85 to 100 % w/w, and desirably from 90 to 100 % w/w, of the total diamine moieties used.

It is possible to include polymer modifying moieties that provide both amino and hydroxyl functionality (i.e. heterodivalent), which will generate both urethane and urea linkages in the product polymer, and examples include mono- and di-ethanolamine and propanolamine, 2-amino-2-methyl-1-propanol, 2-amino-1-butanol, 4-amino-1-butanol, 2-amino-2-ethyl-1,3-propanediol, AMPD(2-amino-2-methyl-1,3-propanediol), 2-amino-2-methyl-1,3-propanediol, and 2-amino-2-hydroxymethyl-1,3-propane-diol. Other suitable materials include the reaction products of hydroxy-acids with di- or polyamines, such as the amidoamine formed from the reaction of 12-hydroxystearic acid with a molar excess of ethylene diamine.

The structuring polymer of the present invention may be branched or cross-linked. "Branched" refers to a polymer which includes a polymeric branch extending from the main backbone of the polymer. The branch may be of essentially any length. "Cross-linked" refers to a polymer wherein at least one branch extending from the main backbone of the polymer is linked to a backbone, generally a backbone which is not a continuation of the backbone the branch originates from, though it could be the same backbone at a distal location. Thus branching could be considered to exist where a branch extends off the main backbone, but the branch is not connected to a backbone at another point, whereas cross-linking occurs when a branch is linked to a backbone at both ends, usually the backbone of a different polymer molecule.

Branching or cross-linking can occur for a number of reasons. As mentioned above, it can occur where a trivalent trimer fatty acid containing moiety is included in the polymer. Branching or cross-linking may also occur where a trivalent polymer modifying moiety is included, e.g. a triol or triamine. Additionally, branching or cross-linking may occur where a trivalent isocyanate is present in the polymer. It is clear that a branch could occur where any trivalent moiety or monomer is present. Branching or cross-linking has also been achieved where castor oil (glycerol triricinoleate acid) is incorporated as a polymer modifying moiety.

Alternatively or additionally, branching or cross-linking can occur where excess diisocyanate is present in the reaction forming the structuring polymer. The excess diisocyanate can react, in the presence of suitable catalysts, to form allophanate and/or biuret linkages at urethane or urea linkages respectively. This provides an important mechanism through which branching or cross-linking can be achieved.

Cross-linking within polymers in general is well known. It is generally accepted that cross-linking increases stability of form at high temperatures by providing the material resistance to flow under stress. Cross-linking is possible through the use of tri- and higher-functional monomers, and also by the induction of allophanate and biuret side reactions. Cross-linking can improve gels in a number of ways; better thermal stability, reduction of syneresis, and greater gel transparency. However, excessive cross-linking can lead to loss of thermo-reversible character, reduced oil or solvent solubility and can lead to undesirable physical properties for handling.

In general it is preferred that 50% or less by weight of the structuring polymers in a structured composition are cross-linked.

The structuring polymer of the present invention may be end capped by a capping moiety. The end capping may control the overall molecular weight and/or the extent of branching and/or cross linking to avoid producing intractable and/or oil-insoluble polymers. Typically the capping moiety is a monovalent molecule which attaches to a terminal group of the polymer (or of a branch), thus preventing further chain extension. The addition of a capping moiety may also modify the polarity of the polymer end groups; this affects the polarity and other characteristics of the polymer. The monovalent capping moieties may comprise any suitable active group, typically amine or hydroxyl, though other active groups, such as carboxylic acid or isocyanate may be suitable.

The structuring polymer typically has at least two urethane and/or urea linkages; suitably three or more urethane and/or urea linkages. Advantageously the polymer has at least two urethane linkages. It is preferred that the polymer comprises more urethane linkages than urea linkages, and accordingly it is preferred that more of the fatty acid of dimer/trimer containing moieties comprise hydroxyl groups than amine groups. In a preferred embodiment the structuring polymer comprises urethane and urea linkages in a mole ratio of 7:3 or greater, more preferably 8:2 or greater, especially 9:1 or greater. It is envisaged that certain preferred embodiments of the polymer may comprise essentially no urea linkages. Polymers which have the high proportions of urethane linkages, as set out above, tend to allow formation of gelled compositions with desirable properties, such as tactile, stability and aesthetic qualities.

Certain embodiments of the structuring polymer may comprise from 2 to 100 urethane or urea linkages. Preferred embodiments comprise from 2 to 50 urethane or urea linkages, especially from 2 to 30 urethane or urea linkages. As mentioned above, the majority of these linkages may be urethane linkages in certain preferred embodiments.

Certain embodiments of the structuring polymer comprise from 2 to 50 fatty acid trimer/dimer containing moieties. Preferred structuring polymers comprise between 2 and 25 fatty acid dimer/trimer containing moieties, especially from 2 to 15 fatty acid dimer/trimer containing moieties.

The melting point of the structuring polymer is typically from 40 to 180 °C, preferably from 60 to 140 °C, more preferably from 90 to 140 °C.

The softening point of the structuring polymer is typically from 30 to 160 °C, preferably from 50 to 120 °C, more preferably from 80 to 120 °C.

The polymers of the present invention are generally soluble in oils whose ClogP values are >0, such as mineral oils, fatty and aromatic alcohols, fatty acids, ester oils, and vegetable oils, and are typically insoluble in water.

The polymers generally have a molecular mass of from 1000 to 100,000 Da, more usually 1500 to 10,000 Da, particularly 2000 to 8000 Da.

A preferred embodiment of the structuring polymer of the present invention comprises the following constituents in the approximate relative quantities indicated in molar equivalents:

1)

$$DI = N$$

2)

$$(N-1) \leq (D+d) \leq (N+1)$$

3)

$$EC1 = (DI - D - d + 1)$$

4)

$$d < D$$

where,
N = from 2 to 10 mole equivalents, preferably 3 to 5
DI = mole equivalents of diisocyanate
D = mole equivalents of fatty acid dimer/trimer containing moiety
d = mole equivalents of polymer modifying moiety
EC1 = mole equivalents of a capping moiety which reacts (or is reacted) only with the diisocyanate moiety

N can be adjusted to suit a particular organic phase/fragrance component.

D, d and DI are understood to be predominantly difunctional, or in any case having an average functionality of 2. When one or more component deviates significantly from this value, the sum of mole equivalents of reactive groups from D, d and EC1 should normally exceed the mole equivalent of reactive groups from DI, such that the final polymer contains substantially no free isocyanate groups.

Such a structuring polymer is particularly suitable for structuring an organic phase with a high loading of fragrance component, or wherein the organic phase consists exclusively of fragrance component. This type of organic phase is typical of air fresheners, particularly concentrated gel air fresheners. When structuring such organic phases, typically the structuring polymer is included in quantities from 10 to 50 % w/w of the total structured composition, typically from 20 to 30 % w/w. The structured composition produced is typically transparent. Additionally, the structured composition will generally have good tactile properties, and will typically be non-greasy, oily or tacky.

In preferred embodiments the constituents of the structuring polymer are as follows:

- Fatty acid dimer/trimer moiety comprising from 90 to 100 % w/w fatty acid dimer diol.
- Diol polymer modifying moiety having a $C_2$ to $C_{10}$ main chain.
- Diisocyanate having a $C_2$ to $C_{10}$ main chain.
- Capping moiety being a monovalent alcohol having a $C_{12}$ to $C_{22}$ main chain.

In an especially preferred embodiment the constituents of the structuring polymer are as follows:

- Dimer diol, e.g. Pripol™ 2033, ex. Croda (97% w/w dimer diol)
- 1,4-butanediol
- Hexamethylenediisocyanate (HDI)
- Isostearyl alcohol e.g. Prisorine™ 3515 ex Croda

In an alternative preferred embodiment the structuring polymer of the present invention comprises the following constituents in the approximate relative quantities indicated in molar equivalents:

1)

$$DI = N$$

2)

$$(D + d) = N + 1$$

3)

$$0 \leq EC2 \leq 2$$

4)

$$d < D$$

where,
N = from 2 to 10 mole equivalents, preferably 3 to 5
DI = mole equivalents of diisocyanate
D = mole equivalents of fatty acid dimer/trimer containing moiety
d = mole equivalents of polymer modifying moiety (when present)
EC2 = mole equivalents of capping moiety which reacts (or is reacted) only with the dimer/trimer-containing moiety and the polymer modifying moiety (when present)

D, d and DI are understood to be predominantly difunctional, or in any case having an average functionality of 2. When one or more component deviates significantly from this value, the sum of mole equivalents of reactive groups from D and d should normally exceed the mole equivalent of reactive groups from DI, such that the final polymer contains substantially no free isocyanate groups.

Such a structuring polymer is particularly suitable for structuring an organic phase comprising a comparatively low amount of fragrance component, e.g. 0.1 to 10 % w/w, typically 2 to 5 % w/w. The remainder of the organic phase may be on oil, typically a mineral oil or ester oil. This type of organic phase is typical of a gel candle. When structuring such organic phases, suitably the structuring polymer is included in quantities from 5 to 45 % w/w of the total structured composition, typically from 15 to 40 % w/w. The structured composition produced is typically transparent.

In preferred embodiments the constituents of the structuring polymer are as follows:

- Fatty acid dimer/trimer moiety comprising 90 to 100 % w/w fatty acid dimer diol.
- Diol polymer modifying moiety having a $C_2$ to $C_{10}$ main chain.
- Diisocyanate having a $C_2$ to $C_{10}$ main chain.
- Capping moiety being a monovalent fatty acid having a $C_{12}$ to $C_{22}$ main chain.

In an especially preferred embodiment the constituents of the structuring polymer are as follows:

- Dimer diol, e.g. Pripol™ 2033 ex. Croda (97% w/w dimer diol)
- 1,4-butanediol and/or ethylene glycol
- Hexamethylenediisocyanate (HDI)
- Isostearic acid, e.g. Prisorine™ 3501 ex Croda

In a general sense, preferred structuring polymers of the present invention have been found to occur wherein 90 % w/w or greater of the fatty acid trimer/dimer moieties are fatty acid dimer diol moieties, which are polymerised with hexamethylene diisocyanate (HDI). More preferably the polymers are end capped with a fatty alcohol or fatty acid, especially isostearyl alcohol or isostearic acid. Optionally the polymer may comprise from 0.5 % w/w to 5 % w/w butanediol (BDO) as a polymer modifying moiety.

The structuring polymer is preferably partially branched or cross-linked. Preferably the cross-linked polymer comprises up to 40 mol % of the total structured polymer.

The polymer may comprise from 0.5 % w/w to 20 % w/w castor oil, based on the total fatty acid dimer/trimer containing moiety content of the polymer. The trifunctional castor oil leads to cross-linking and/or branching, typically via urethane linkages.

In one preferred embodiment of the present invention there is provided a polymer formed from a fatty acid dimer diol (Pripol™ 2033, ex Croda - 2% monomer, 96.5% dimer, 1.5% trimer) and hexamethylene diisocyanante (HDI), end capped with isostearyl alcohol (iC18, Prisorine™ 3015, ex. Croda). The isostearyl alcohol is used in short measure, so after the urethane reaction a part of the molecules is isocyanate end-capped. Free isocyanate may be allowed to react with the secondary H of the urethane forming an allophanate linkage.

Although it is impossible to predict the exact structure of the polymer, it is expected that an approximate average, or representative structure would be:

For a non-branched or non-cross-linked polymer-

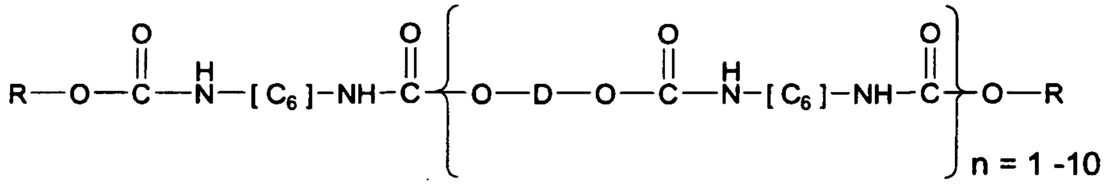

where

- R is the chain of an end cap, preferably the alkyl chains from isostearyl alcohol, and
- D is the alkyl portion of the dimer diol fatty acid containing moiety.

For a cross-linked polymer-

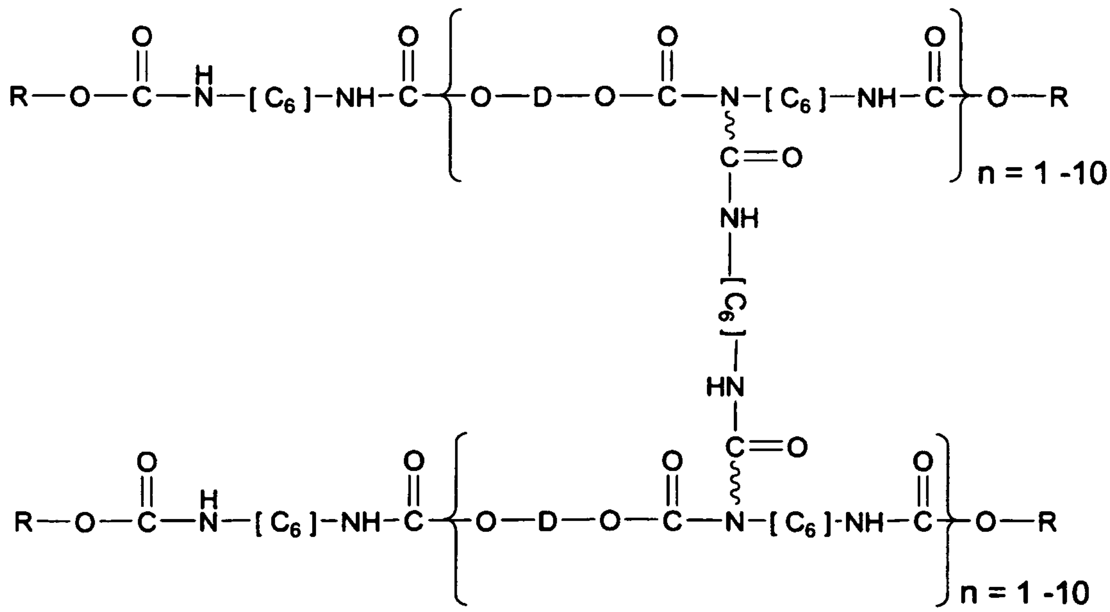

Where R and D are as defined above.

The wavy linkage indicates that the number and position along the polymer chain of the allophanate cross-links is variable. An alternative structure giving branching can be formed, e.g. when an isocyanate-terminated fatty acid dimer/trimer containing moiety reacts into the backbone of the polyurethane structuring polymer.

In another preferred embodiment of the present invention there is provided a polymer formed from dimer diol (Pripol™ 2033, ex Croda), hexamethylene diisocyananate (HDI), 1,4 butanediol (BDO), castor oil, end capped with isostearyl alcohol (iC18).

In the above embodiment the cross-linked polymer would likely comprise about 5-10 mol% of the total polymer, calculated relative to the non-cross-linked polymer. When added to an organic phase at 20% w/w, both the above polymers give (almost) completely transparent gels when gelling both apolar and relatively polar fragrances. Both types perform better in more apolar fragrances, where they can be maintained at a temperature of 60°C and sometimes higher, without showing creep. For the polar fragrances the temperature limit of stability to creep is around 50°C.

The structuring polymers described above have demonstrated excellent abilities to gel polar and non-polar fragrance compositions, with generally high levels of translucence/clarity.

According to another aspect of the present invention there is provided a structured composition comprising an organic phase comprising a fragrance composition, the organic phase being structured by a structuring polymer, wherein the structuring polymer contains fatty acid dimer/trimer containing moieties and comprises urea and/or urethane linkages.

Preferred embodiments of the structuring polymer are set out above.

In a preferred embodiment the structured composition is a gel. Other forms of structured compositions may be suitable such as waxy compositions or viscous liquids, especially when such compositions are contained in a jar, cup, glass or similar supporting device.

The structuring effect of the structuring polymer may vary depending on the amount of structuring polymer included relative to the organic phase, and may range from slightly increasing in viscosity of the organic phase, to

providing a gel or waxy solid in which the organic phase is essentially completely bound within the matrix of the structuring polymer.

The structuring polymer may be used to modify the organic phase, for example to improve properties such as handling and application, emulsion stability, wetting and adhesion to surfaces, and heat transfer characteristics.

The structuring polymer of the present invention is capable of gelling an organic phase, typically an oil, providing gelled compositions having relatively high yield stresses. This property allows the suspension of solids for visual or functional effect and prevents creep when the gelled material experiences gravitational or other forces such as in a vertical or inclined surface.

Suitably the structured composition is an air freshener, typically a gel air freshener. The air freshener may suitably be mounted on a substrate, e.g. a shaped glass or plastic mould.

Alternatively, (or additionally) the structured composition is a candle, preferably a gel candle.

Alternatively, the structured composition may be a wax, polish, leather care composition, solvent, cleaner, fuel, grease, non-aqueous detergent liquid, lubricant or adhesive.

The structuring polymer can be used in applications where solid particles must be stably suspended in an organic phase. For example, such solid particles may be pigments, abrasive powders, ceramic powders, dielectric materials, minerals, metal powders, carbon black, organic and inorganic salts. Suitable compositions which include such particles include inks, paints and coatings, abrasive cleaners and polishes, ceramic pastes, bitumen and asphalt compositions, soldering pastes and inkjet printer ink.

Generally the structured composition comprises from 0.2 to 60% w/w structuring polymer, more usually from 0.5 to 35% w/w, especially from 1 to 20% w/w of the total composition.

The organic phase may, in some embodiments, consist exclusively of a fragrance composition, e.g. a pure fragrance oil or oils. Alternatively, the organic phase may contain a fragrance composition in association with one or more additional organic compounds; these additional compounds will typically be oils. Typically the oil is one or more of a mineral oil, aromatic oil, other hydrocarbon oil, vegetable oil, fatty alcohol and an ester oil. High concentrations of fragrance composition are typical of organic phases for use in air fresheners.

Typical organic substances that can be structured, e.g. gelled, using the structuring polymer of the invention include liquid and low-melting temperature alcohols including relatively short chain alkanols, such as *t*-butanol and pentanol; medium chain alcohols, such as 2-ethylhexanol and 2-ethyl-1,3 hexanediol; long chain alcohols such as isodecanol, isotridecanol, cetyl alcohol, oleyl alcohol, octyldodecanol; liquid $C_8$ to $C_{32}$ alcohols, e.g. Guerbet alcohols such as Isofol 32 (ex. Sasol); liquid polyols such as glycols and (poly)glycerol; or aromatic alcohols such as benzyl alcohol; polycyclic alcohols such as abietyl alcohol.

Alternatively, organic substances such as branched liquid fatty alcohols may be structured or gelled using the structuring polymer of the invention include octyldodecanol or isostearyl alcohol (see above), e.g. the isostearyl alcohol available from Croda under the tradename Prisorine™ 3515 (HBSP 17.9) and synthetic (petrochemical) branched fatty alcohols such as those sold by Shell Chemicals under the Neodol trademark or by Sasol under the Nafol trademark.

In certain embodiments of the present invention one or more of the following organic substances may be structured or gelled using the structured polymer or the invention:

- polymers of ethylene oxide, propylene oxide, butylene oxide and higher alkylene oxides, and mixtures thereof e.g. Voranol 1010 available from Dow Chemical Company ( polypropylene glycol MW - 1000)
- fatty alcohol polyalkoxylates, particularly propoxylates such as the alkoxylates of $C_{12}$ to $C_{20}$ fatty, particularly $C_{14}$, $C_{16}$ and $C_{18}$ fatty alcohols which can be linear e.g. as in palmitic and stearic acids, or branched e.g. as in isostearyl alcohol (in practice a product typically derived from dimer acid manufacture which contains a mixture of mainly branched $C_{14}$ to $C_{22}$ alcohols averaging about $C_{18}$), with from 3 to 25 particularly from 7 to 20 alkoxylate, especially ethoxylate, propoxylate or mixtures of ethoxylate and propoxylate, units e.g. the stearyl alcohol 15-polypropoxylate available from Croda under the tradename Arlamol E (HBSP 20.8);
- alkoxylated, particularly ethoxylated polyol esters e.g. ethoxylated sorbitan esters such as those sold under the tradename Tween™ by Croda;
- ester oils particularly those based on $C_2$ to $C_{30}$ linear, branched or unsaturated fatty acids and linear, branched or unsaturated fatty alcohols, and typically esters derived from monocarboxylic acid(s) with monohydric alcohol(s); di- or tri-carboxylic acid(s) with monohydric alcohol(s); or di- or poly-hydric alcohol(s) with monocarboxylic acid(s), e.g. the glycerol tris-2-ethylhexanoate ester oil available from Croda under the tradename Estol™ 3609 (HBSP 20.4), the isopropyl isostearate oil available from Uniqema under the tradename Prisorine™ 2021 (HBSP 17.7) the methyl oleate oil available from Croda under the tradename Priolube™ 1400 (HBSP 17.9), methyl caprylate, synthetic triglyceride esters such as glycerol tri-($C_8$ to $C_{24}$)ates e.g. glycerol tricaprylate such as Estasan™ 3596 available from Croda, and glycerol triricinoleate, PEG oleate and isostearate, isopropyl laurate or isostearate, trimethylpropane triesters e.g. with mixed $C_8$/$C_{10}$, stearic or oleic acids; natural triglycerides such as rape seed (canola) oil, soya oil, sunflower oil and fish oil;

- methylated natural triglycerides such as methylated rape seed, soya and/or sunflower oils;
- aromatic ester oils, particularly esters if benzoic acid or phthalic acid and C8 to C18 monohydric alcohol(s) e.g. the $C_{12}$ to $C_{15}$ benzoate oil from Finetex under the tradename Finsolve TN (HBSP 19.1), DOP (dioctyl phthalate) and others;
- branched liquid fatty alcohols, isostearyl alcohol (see above) e.g. the isostearyl alcohol available from Croda under the tradename Prisorine™ 3515 (HBSP 17.9), or synthetic (petrochemical) branched alcohols such as those sold by Shell Chemicals under the Neodol trademark;
- branched liquid fatty acids, particularly isostearic acid and dimer acid (dimerised fatty acids, particularly oleic and/or linoleic acids), such as dilinoleic acid (HBSP 17.8);
- hydrocarbons including toluene, xylene, and liquid paraffinic materials such as hexane, octane, gasoline, diesel, liquid hydrocarbon waxes, lamp oil, paraffinic oils such as Sunspray 6N, 8N and 11N from Sunoco and Puccini 19P from Q8, (iso)-paraffinic oils such as Isopar V and Exxol D140 from ExxonMobil, and aromatic mineral oils such as the alkyl benzenes available from ExxonMobil under the Solvesso brand; and
- miscellaneous liquids such as isophorone (3,3,5-trimethyl-2-cyclohexene-1-one), liquid (at 25°C) fatty acids such as caprylic, isostearic, oleic, and vegetable oil fatty acids, ketones such as methyl ethyl ketone (MEK), aldehydes such as butanal, polyester polyols based on dimer and trimer acids such as the Priplast™ range available from Croda.

The organic phase may, of course, comprise mixtures of two or more different types of organic substances.

As mentioned above the organic phase may consist substantially entirely of one or more "pure" fragrance oils, i.e. the organic phase is comprised substantially entirely of a fragrance oil and no additional "carrier" oil is present. However, fragrance compositions, even those marketed as "pure" fragrance oils, generally contain at least some non-fragrance oil blended with the fragrance.

The term "fragrance" is intended to refer to a chemical, or blend of chemicals having a desired odour. Fragrances are commonly used, and any known fragrance or blend of known fragrances may be used in accordance with the present invention. In particular naturally occurring or synthetic fragrances may be used. The fragrance for use in the structured composition may be any fragrance material known and used in products such as perfumes, soaps, detergents and the like. International Flavours & Fragrance (IFF New York, N.Y.) sell a large number of suitable materials, for example Aphermate, Andrane, Clonal, Diola, and Fraistone (all trademarks).

In certain embodiments the fragrance composition may contain an alcohol based solvent such as Dowanol TPM, benzyl alcohol or carbitol. Alternatively the solvent may be an oil based solvent, in particular a paraffin based solvent such as Isopar V or Isopar M. In a further embodiment the solvent may be isopropyl myristate or (di)propylene glycol.

The nature and amount of the fragrance composition included in the structured composition of the present invention is dependent on the desired intensity of the fragrance and the desired degree of fragrance emission from the structured article. The suitable nature and amount of fragrance to be included in the gelled article may be determined by one skilled in the art. However, generally the structured composition comprises at least 2 % w/w fragrance composition based on the total weight of the structured composition.

The structuring polymer should, of course, be compatible with the fragrance composition. The structure of the structuring polymer is therefore generally dependent on the nature and structure of the fragrance composition, though it should be understood that structuring polymers according to the present invention are in general stable with a range of different fragrances. In particular, the nature of the terminating groups of the structuring polymer to achieve effective structuring are typically dependent on the nature and structure of the fragrance composition.

The structure of the structuring polymer of the present invention can be modified in a controlled and predictable manner by modifying the polymerisation reaction used to form the structuring polymer. In particular, the structure of the reactants used to form the structuring polymer, and/or the reaction conditions used to form the structuring polymer may be altered. Alternatively, the termination groups of the structuring polymer may be modified through a capping step as described above. Accordingly, the structure of the structuring polymer may be altered to ensure compatibility with a wide range of different fragrance compositions. As such the structuring polymer of the present invention may have broad utility across a wide variety of fragrance compositions, which is a significant advantage over current structuring polymers which are highly restricted to particular species of fragrance composition.

Prior art structuring polymers generally suffer from an inability to structure both polar and non-polar (apolar) fragrance compositions. Fragrance compositions can be either polar or non-polar, and this greatly affects their compatibility with prior art structuring polymers. Prior art structuring polymers are typically able to gel only polar or apolar fragrance compositions, or even only fragrance compositions within a certain narrow range of polarity (polarity should be considered as a continuum). Where such structuring polymers are used to gel compositions outside the preferred polarity range, typically the gelled system becomes opaque and also may become unstable, i.e. may lose structure or become oily at low temperatures or after little time, or in fact may not form a gel at all.

Structuring polymers according to the present invention, particularly in certain preferred embodiments, are able

to stably gel both polar and non-polar fragrance compositions. This is highly advantageous as it means a single gellant can be used for a variety of different fragrances.

The CLogP (partition coefficient in octanol/water) is widely used to determine the polarity of substances and is well understood in the fragrance and personal care industry. It also has the advantage that values of pure materials (and mixtures thereof) can be calculated using software programmes, rather than measured experimentally.

Preferred embodiments of the present invention are able to gel fragrance compositions having a polarity of from -3 to +6 according to the ClogP scale, more preferably from -1 to +5.

Optionally, an inert co-solvent and/or plasticiser can be combined with the structuring polymer to improve handling properties and/or reduce melting temperatures of the structuring polymer. The rheological properties of the structured composition can also be modified by addition of co-solvents, and this can be used to modify the rheological properties of the structured composition. Examples of co-solvents which are especially effective in reducing melting range include, 1-phenoxy-2-propanol, 3,7-dimethyl-6-octen-1-ol beta citronellol, 3,7-di-methyl-2,6-octadien-1-ol, 3-hexane-1-ol, cyclohexanone, tetramethyl urea, ethylene glycol monopropyl ether, 2-ethyl-hexanol, 1-pentanol, propylene glycol monopropyl ether, 2,4,4-trimethyl-1-pentanol, cyclo-hexanol, hexyl alcohol, and ethylene glycol monoisoproyl ether. When used, the amount of co-solvent will generally be used at a proportion of between 10 and 90%, and more usually from 40 to 75% by weight based on the structuring polymer, representing from 0.5 to 45%, more usually 1 to 10% by weight of the overall formulation.

When the co-solvent is correctly selected, the softening point can be reduced substantially, in some instances to the point that the polymer solution is at room temperature. As such, the gelled articles of the present invention may generally be manufactured at temperatures substantially below the softening point of the neat polymer, typically between 20 and 60°C, reducing the cost and inconvenience of manufacture, and allowing the incorporation of heat sensitive components, in particular fragrance compositions, without risk of damage. It is a surprising property of the structuring polymers of the present invention that they can be processed at such low temperatures, yet form a stable structured composition which may, e.g. resist creep, at comparatively high temperatures. Structured compositions comprising prior art structuring polymers generally require processing temperatures close to the melting point of the structuring polymer, typically 80 to 120°C.

Other components such as antioxidants, essential oils, preserving agents and similar materials may also be included in the structured composition.

Colours, dyes, pigments, small decorative articles and icons and/or insect repellents may optionally be included in the structured composition.

The structured compositions of the present invention are generally clear or translucent. The terms "clear", "clarity", "transparent" and "translucent" are intended to have their ordinary dictionary meanings. As such, a clear gel allows viewing of objects behind it, whereas a translucent gel causes light to be scattered whilst allowing light to pass therethrough.

There are various degrees of transparency and translucency ranging from absolutely clear to hazy. The percentage haze associated with a composition may be determined by shining a white light through a sample of the composition having a pre-determined thickness at room temperature and determining the diffuse transmittance and total transmittance of the light. The percentage haze may be calculated according to the equation:

$$\% \text{ haze} = (\text{diffuse transmittance}/\text{total transmittance}) \times 100.$$

Typically the structured composition is substantially transparent. Generally the gelled article allows at least 50% of visible light incident upon it to travel through it; generally at least 60%; preferably at least 70% of visible light incident upon it.

Suitably the structured composition of the present invention has an associated percentage haze value of less than 50%; more suitably less than 30%; advantageously less than 10%.

Preferably the structured composition is in the form of a stiff gel with good tactile properties. The structured composition is preferably relatively non-greasy and relatively non-oily. For example, a non-greasy or non-oily composition can be touched by a user and they will not receive any appreciable oily or greasy residue. Alternatively, when an absorbent substance, such as tissue paper, is placed in contact with the composition, organic phase does not seep into the absorbent substance. Additionally, preferably the structured composition preferably does not feel tacky when touched by a user.

The structured composition, especially a gelled composition, may suitably be sufficiently rigid to support itself. Advantageously the structured composition is self-supporting in that it retains its shape at room temperature in the absence of shear. The structured composition is generally self-supporting if its yield value is greater than the sheer stress imposed by gravity. The structured composition may suitably be in the form of a free standing article such as a free standing pillar or column.

Alternatively the structured composition may be provided in a supporting device such as a container.

Advantageously the structured composition does not creep or flow over time. Typically, upon application to a vertical surface (such as a glass plate), the structured composition exhibits no evidence of creep over one month, this is thought to be due to the yield stress of the polymer.

Suitably a gelled composition in accordance with the present invention is stable in that it does not spontaneously, or over time, undergo syneresis and separate into a liquid and a gel (or "bleed"), and does not spontaneously form a colloidal suspension due to contraction of the gel.

It is desirable that the structured composition should remain stably structured at ambient temperature for at least 1 month, and at elevated temperatures, typically up to at least 50°C and desirably up to 60°C, for at least 2 weeks, and at sub-ambient temperatures usually at least as low as 0°C for up to eight weeks. Typically the structured composition of the present invention has high thermal stability when subjected to these conditions, or periodic cycling between these conditions e.g. to simulate daily ambient conditions in extreme climates.

The structured composition may comprise an exterior coating, typically a solid exterior coating. The coating can be, for example, a polyamide resin or a styrene acrylate resin. According to one embodiment, the structured composition is activated and may start emitting fragrance, upon removal, or partial removal, of the exterior coating. Alternatively, a semi-permeable coating or container can be used. Another function of such coatings may be to prevent the user contacting directly the gel, which may give a greasy, oily, waxy or tacky sensation; although this is generally not required with structured compositions of the present invention which have good tactile properties.

In one embodiment, the structured composition may be in the form of a scented candle intended for decorative purposes. Such a candle would generally comprise a wick, and the structured composition may be activated, and may start emitting fragrance, upon lighting of the wick. The structured composition contains and emits a fragrance and the candle is thus typically in the form of a fragranced or scented candle. Generally, the wick is lit melting the structuring polymer of the structured composition to form a warm pool of molten liquid around the wick, and fragrance is then released from this warmed pool.

According to a further aspect of the present invention there is provided a method of forming a structuring polymer as described herein, the method comprising the steps of:

- providing fatty acid dimer/trimer containing moieties; and
- reacting the fatty acid dimer/trimer containing moieties with a suitable molecule to form a polymer comprising urea and/or urethane linkages.

Suitably the fatty acid dimer/trimer containing moieties are reacted with an isocyanate molecule, preferably an diisocyanate molecule.

Generally the fatty acid dimer/trimer containing moieties and isocyanate are polymerised to form the polymers of the present invention in the presence of a catalyst. Catalysts for urethane or urea polymerisation reactions can be tertiary bases, e.g. bis-(N,N'-dimethylamino)-diethyl ether, dimethylaminocyclohexane, N,N-dimethylbenzyl amine, N-methyl morpholine, reaction products of dialkyl-(b-hydroxyethyl)-amine with monoisocyanates, esterification products of dialkyl-(b-hydroxyethyl)-amine and dicarboxylic acids, and 1,4-diaminobicyclo-(2.2.2)-octane, and non-basic substances such as metal compounds e.g. iron pentacarbonyl, iron acetyl acetonate, tin(II) (2-ethylhexoate), dibutyl tin dilaurate, molybdenum glycolate, stannous octoate, TBT and TIPT. Advantageously the catalyst is di-butyl stannous dilaurate or stannous octoate.

Suitable catalysts to catalyse the formation of cross-liking or branching via allophanate or biuret linkages include stannous octoate, potassium carbonate (Dabco k15, ex. Air Products) and triethylamine (Dabco TMR, ex. Air Products)

It has been found to be practical to carry out the synthetic reactions without solvent or diluent using the raw materials neat. In particular, reagents such as monocarboxylic acid esters included as reagents to form capping moieties can act also as reaction diluents/solvents until they are incorporated into the polymers. However, as mentioned above, it is possible to use co-solvents or diluents if desired to improve the ease of handling of the resultant structuring polymer. Generally any liquid or low melting point solid which is substantially inert to the reaction mixture, can be safely handled, and which can solubilise the structuring polymer and its constituents such that the desired handling improvement are achieved will be suitable. Suitable co-solvents or diluents include acetone, toluene, plasticiser esters, aromatic esters such as benzoates (e.g. 2-ethylhexyl benzoate), or isopropyl esters such as isopropyl myristate, triglycerides such as glycerol trioleate, N-methylpyrrolidone, and dialkyl carbonates. Examples of co-solvents, suitable for adding to the formed structuring polymer, which are especially effective in reducing melting range include, 1-phenoxy-2-propanol, 3,7-dimethyl-6-octen-1-ol beta citronellol, 3,7-di-methyl-2,6-octadien-1-ol, 3-hexane-1-ol, cyclohexanone, ethylene glycol monopropyl ether, 2-ethyl-hexanol, 1-pentanol, propylene glycol monopropyl ether, 2,4,4-trimethyl-1-pentanol, cyclo-hexanol, hexyl alcohol, and ethylene glycol monoisoproyl ether.

The method may comprise the step of capping at the polymer with a capping moiety. This step may be carried out after the polymerisation step, or at the same time. The capping moiety is typically a monovalent alcohol, amine, acid,

isocyanate or ester. The end cap to be reacted will, of course, be dependent on the group at the end of the polymer.

Suitably the capping step includes esterification of terminal hydroxyl groups of the structuring polymers with fatty acids or fatty methyl esters, or isocyanate groups at the end of the structuring polymer may be capped with an alcohol. Typically the capping step may comprise esterification of the terminal groups of the structuring polymer.

Polymeric capping moieties can be made prior to polymerisation, for example by reacting fatty acid dimer/trimer containing moieties with a suitable capping molecule (e.g. fatty acid or alcohol) to form the desired terminating groups.

Alternatively, the capping moieties can be added post polymerisation through reaction of the part-formed structuring polymer with capping moieties. Where the part-formed structuring polymer is isocyanate ended, the method typically includes a capping step wherein the capping group is an alcohol and will give a substituted urethane ended polymer.

Where the polymer is hydroxyl (diol) ended, the capping reaction may be:

- with an alcohol (or a reactive derivative), under etherification conditions, particularly in the presence of an etherification catalyst such as potassium carbonate, potassium hydroxide, sodium hydroxide or calcium hydroxide,
- or a monoisocyanate including silicone-functionalised compounds such as 3-isocyanatopropyl-trimethoxysilane, available under the Silquest trademark.
- or a carboxylic acid (or a reactive derivative) under esterification conditions, particularly in the presence of an esterification catalyst such as tetrabutyl titanate (TBT), tetra-isopropyl titanate (TIPT), stannous octoate e.g. the commercial product Tegokat 129, bases e.g. potassium or sodium carbonate, acids e.g. para-toluene sulphonic acid (PTSA), dodecyl benzene sulphonic acid (DBSA) or sulphuric acid, more particularly by reacting with an ester of the formula $R^1COOR^2$, where R is as any hydrocarbyl group, particularly a $C_1$ to $C_{60}$ group, more usually a $C_1$ to $C_{44}$, especially an alkyl, group, and $R^2$ is a lower, particularly $C_1$ to $C_8$, alkyl and especially a methyl, group under transesterification conditions, particularly in the presence of transesterification catalyst such as TBT, TIPT, stannous octoate, or a base e.g. potassium or sodium carbonate.

Suitably the capping moieties are added to the reaction mixture prior to polymerisation but are inert during polymerisation, and act to improve processing and handling. Typically such capping groups act as diluents, thinners and/or solvents during the polymerisation reaction. Such capping moieties include alkyl esters.

Generally where the part-formed structuring polymer is (or would be) hydroxy or amine ended, the reaction will generally be carried out in two stages, first formation of the part-formed structuring polymer, and then capping the part-formed polymer (if desired). Where the part-formed structuring polymer is (or would be) isocyanate ended, and particularly where the capping groups are hydroxyl compounds (alcohols) or amines, the reaction may be carried out in a single step with all the reagents in a single vessel from the outset.

Reactions with isocyanates, polymerisation or capping reactions, are generally carried out at temperatures from 50 to 160 °C, more usually 60 to 140 °C. Reactions with acids or esters to form ester or amide end caps with acids are generally carried out at temperatures from 110 to 270 °C, more usually 180 to 230 °C, e.g. at about 225 °C. Both direct and trans-esterification and amidation reactions can be carried out at ambient pressure or at moderate vacuum, e.g. from 600 to 10 mBar abs (60 to 1 kPa abs) will usually be used. Inert gas, e.g. nitrogen, may be sparged through the reaction mixture under ambient or reduced pressure to aid removal of volatiles from the reaction, and to reduce discoloration of the polymer due to contact with air. Generally, a small excess of the acid or the ester (usually a methyl ester) will be used.

According to a further aspect of the present invention there is provided a method of forming a structured composition comprising an organic phase comprising a fragrance composition, the method comprising the steps of:

- providing a structuring polymer which contains fatty acid dimer/trimer containing moieties and comprises urea and/or urethane linkages;
- providing an organic phase comprising a fragrance composition; and
- combining the structuring polymer with the organic phase under suitable conditions to form a structured composition.

Details of structuring polymers and organic phases for use in the method are set out above. Suitably the structured composition formed according to the method of the present invention is as described above. In particular it may be an air freshener or a gel candle.

The structuring polymer is typically combined with the organic phase at moderately elevated temperature, typically from 50 to 140 °C, more usually from 60 to 120 °C, commonly from 80 to 110 °C. The resultant mixture is then typically cooled through a cooling step or by allowing the resultant mixture to cool to ambient temperature. The structuring effects typically become apparent on cooling. It has been found that the cooling rate can influence the properties of the structured composition, and can be used as a method to control the processing and finished properties.

Alternatively, the structuring polymer may be combined with the organic phase at temperatures close to ambient

temperatures, typically at temperatures of 20 to 40 °C, preferably 20 to 25 °C. Preferably, when the structuring polymer is combined with the organic phase at or near ambient temperature combination takes place in the presence of a co-solvent to improve handling properties, suitable co-solvents are mentioned above.

The fragrance composition may be mixed with the structuring polymer at any time prior to formation of the gel. Having regard to the volatile nature of many fragrance compositions, the fragrance composition is generally added at relatively low temperatures during the method of forming the structured composition. Where the method involves the application of elevated temperatures upon combination of structuring polymer and the organic phase, the fragrance composition may be added to the organic phase during the cooling step. Where the structuring polymer, co-solvent and the organic phase are combined at temperatures close to ambient temperatures, the fragrance composition may be added at any time from mixing of the structuring polymer, co-solvent and the organic phase, i.e. it may be in the organic phase from the start.

Where the structured composition is a scented candle, the method may include the steps of transferring the mixture comprising the structuring polymer and organic phase to a mould, and optionally positioning a wick in the mixture and allowing the mixture to cool. The candle may be removed from the mould following cooling, or the mould may provide support for the candle during use.

According to a further aspect of the present invention there is provided the use of a structuring polymer which contains fatty acid dimer/trimer containing moieties and comprises urea and/or urethane linkages, in the manufacture of a structured composition comprising an organic phase which comprises a fragrance composition, especially an air freshener.

The present invention will now be further described by way of non-limiting examples, with reference to the accompanying drawings in which; Figures 1, 2 and 3 show the fragrance release curves of three different fragrances (frg 1, frg2 and frg 3 repectively) in air freshners made from polymer SE32 as described below.

The following materials are referred to in the examples below. All parts and percentages are by weight unless otherwise stated.

Materials

Fatty Acid Dimer/Trimer Containing Moieties

| | |
|---|---|
| DD1 | dimer diol (2% monomer, 96.5% dimer 1.5% trimer) - Pripol™ 2033 ex Croda |
| DD2 | trimer triol (polyol derived from a technical quality trimer acid and containing 2% monomeric, 54.5% dimeric and 43.5% trimeric alcohol) |
| DD3 | hydroxyl ended oligomer from reaction of dimer acid and ethylene glycol |
| DD4 | dimer diamine |
| DD5 | hydroxyl ended oligomer from reaction of dimer acid and dimer diol, Priplast™ 3197 ex Croda |
| DD6 | trimer acid, Pripol™ 1040 ex Croda |
| DD7 | dimer acid, Pripol™ 1017 ex Croda |
| DD8 | hydroxyl ended oligomer from reaction of dimer acid and 1,6-hexane diol, Priplast™ 3196 ex Croda |

Polymer Modifying Moieties - Diols

| | |
|---|---|
| D1 | 1,6-hexanediol (MP 39-42°C) |
| D2 | 1,4-butanediol |
| D3 | 2-ethyl-hexane-1,3-diol |
| D4 | isosorbide |
| D5 | bisphenol- A 2.2 propoxylate |
| D6 | ethylene glycol |
| D7 | trimethylol propane |

Polymer Modifying Moieties - Diamines or Mixed Amine/Hydroxyl

A1 monoethanolamine
A2 ethylene diamine

Isocyanates

| | |
|---|---|
| IC1 | Hexamethylene diisocyanate (HDI), Desmodur H ex Bayer |
| IC2 | HDI-trimer, Desmodur N3600 ex Bayer |
| IC3 | dicyclohexanemethane-4,4'-diisocyanate, Desmodur W ex Bayer |
| IC4 | modified MDI, Desmodur CD ex Bayer |
| IC5 | 3-isocyanatopropyl-trimethoxysilane, Silquest ex GE Silicones |

Capping Moieties

| | |
|---|---|
| Est1 | methyl isostearate, Prisorine™ 3760 ex Croda |
| Est2 | methyl stearate |
| Est3 | methyl oleate, Priolube™ 1400 ex Croda |
| Est4 | methyl benzoate |
| Est5 | isopropyl myristate, Estol™ 1514 ex Croda |

| | |
|---|---|
| Acd1 | Stearic acid, Prifac™ 2979 ex Croda |
| Acd2 | Myristic acid, Prifac™ 2942 ex Croda |

| | |
|---|---|
| Alc1 | stearyl alcohol |
| Alc2 | isostearyl alcohol |
| Alc3 | Branched C32 alcohol, Isofol 32 ex Sasol |
| Alc4 | Branched C20 alcohol, Isofol 20 ex Sasol |
| Alc5 | Propylene glycol monoisostearate, Prisorine™ 2034 ex Croda |

Ami1 tallow amine

Urethane/Urea Reaction Catalysts

| | |
|---|---|
| UC1 | di-butyl stannous di-laurate |
| UC2 | stannous octoate |

Esterification/Transesterification Catalysts

| | |
|---|---|
| TBT | tetrabutoxytitanate $[Ti(O\text{-}n\text{-}C_4H_9)_4]$ as 20 wt% solution in dioctyl azelate |
| SO | stannous octoate, Tegocat 129 ex Goldschmidt |

Oils

| | |
|---|---|
| Oil1 | Paraffin (highly liquid) - ex Merck |

Fragrances

| | |
|---|---|
| Frg1 | Fragrance FG (Apolar fragrance ex Givaudan) |
| Frg2 | Fragrance BB (Polar fragrance ex Givaudan) |
| Frg3 | Fragrance Me-BB (Polar fragrance with no free alcohol groups, ex Givaudan) |

Solvents

| | |
|---|---|
| CoS1 | Dowanol TPM, ex Dow Chemicals |
| CoS 2 | Benzyl alcohol |
| CoS 3 | Carbitol |
| CoS 4 | DPG (dipropylene glycol) |
| CoS 5 | Isopar V, ex Exxon Mobil Chemicals |
| CoS 6 | Isopar M, ex Exxon Mobil Chemicals |
| CoS 7 | Isopropyl myristate, Estol™ 1514, ex Croda |

Examples

Example 1 - Preparation of an ester-terminated polyurethane structuring Polymer by "pre-esterification" route.

Dimer diol (DD1) (515.30 g; 0.95 mol), flaked stearic acid (Acd1) (111.47 g; 0.39 mol), flaked myristic acid (Acd2) (90.22g; 0.39 mol), dimer acid (DD7)(48.03g; 0.079 mol) and if desired 1,4-butanediol (D2) (37.58g; 0.42mol) and/or flaked trimethylol propane (D7) (13.43 g; 0.10 mol) were charged to a 2l flanged flask ("reactor") equipped with an external electrical heater, nitrogen inlet, thermometer, condenser and receiving vessel, central stirrer and addition port. The mixture was rapidly heated to 225°C under nitrogen sparging through the mixture, and TBT (440 μl) dosed. Vacuum was applied 450-350 mbar, and the mixture held at 225°C under vacuum until the acid value was 0.1 mg (KOH).g-1or less. The mixture was cooled down to 125°C, nitrogen sparging stopped, and full vacuum (20-10 mbara) was applied for 1 hour to remove water from the mixture. The mixture was then cooled down to 60°C, while vacuum was relieved with nitrogen sparging over the mixture. In the case that the 1,4 butanediol and/or trimethylol propane was not added before the pre-esterification, these components were added now through the addition port. At ca. 70°C catalyst UC1 (400 μl) was added and hexamethylene diisocyanate (IC1) (162.69 g; 0.97 mol) dosed through the addition port at a rate of 150 $g.h^{-1}.kg^{-1,}$ and the mixture heated at a rate of 50°C per hour to a temperature of approximately 140°C. The mixture was kept for 2-3 hours until the hydroxyl value fell to 10 $mg(KOH).g^{-1}$ (or less). The product was discharged and allowed to cool to ambient temperature to yield the polymer as a slightly yellow hazy waxy solid.

The advantage of this processing route is the flexibility and customisation it offers in the choice of polymer end-groups; fatty acids are cheap and available in many different purities, and with alkyl chain lengths from $C_8$ to $C_{22}$ and higher.

Example 2 - SE1 : Preparation of an ester-terminated polyurethane structuring polymer by "cost-esterification" route.

Dimer diol (DD1) (515.3 g; 0.95 mol), 1,4 butanediol (D2) (37.58 g; 0.42 mol), flaked trimethylol propane(D7) (13.43 g; 0.10 mol), molten methyl stearate (Est1) (117.02 g; 0.39 mol) and isopropyl myristate (Est5) (105.96 g; 0.39 mol) were charged to a 2l flanged flask ("reactor") equipped with an external electrical heater, nitrogen inlet, thermometer, condenser and receiving vessel, central stirrer and addition port. The mixture was heated under an inert nitrogen atmosphere (maintained throughout the reaction) to ca. 70°C. Catalyst (UC1) (400 μl) was then added and hexamethylene diisocyanate (IC1) (162.69 g; 0.97 mol) added through the addition port using a dosing pump at a rate of 150 $g.h^{-1}.kg^{-1}$.

The mixture was heated at a rate of 50°C per hour to a temperature of approximately 180°C, and dimer acid (DD7) (48.03 g; 0.079 mol) was dosed. The catalyst SO (0.8 g) was added and the mixture held at 180°C until the hydroxyl value fell to 10 mg(KOH).$g^{-1}$ (or less). The reaction mixture was allowed to cool to 140-150°C under nitrogen sparge and the product discharged and allowed to cool to ambient temperature to yield the oligomer as a slightly yellow hazy waxy solid..

In this example, the fatty ester added at the beginning of the reaction acts as a solvent and diluent, which offers processing and handling advantages.

Example 3 - SE2 : Preparation of a urethane-terminated polyurethane structuring poymer.

Dimer diol (DD1) (567.16 g; 1.04 mol) and isostearyl alcohol (Alc2) (199.46 g; 0.74 mol) were charged to a reactor as described in Example 1 and heated to 70°C. Catalyst (UC1) (400 μl) was then added followed by diisocyanate (IC1) (233.38 g; 1.39 mol) added through the addition port as described in Example 1 (during isocyanate dosing the viscosity increases and therefore it can be desired to heat the mixture to keep it liquid enough in case the exothermic energy is not enough to keep the mixture liquid). The mixture was heated at 50°C per hour up to 140°C and held for 2-3 hours after which the hydroxyl value was <5 mg(KOH).$g^{-1}$. The product was discharged and allowed to cool to ambient temperature to yield the oligomer as a white rubbery translucent solid.

Example 4 - SE3 : Preparation of an ester-terminated Polyurethane structuring Polymer by "post-esterification" route

This example includes the use of a higher functional polyol as component of the base polymer.

Trimer triol (DD2) (613.6 g; 0.94 mol), methyl stearate (Est2) (189.1 g; 0.65 mol) and stearyl alcohol (Alc1) (44.9 g; 0.17 mol) were charged to a reactor as described in Example 1. The mixture was heated under an inert nitrogen atmosphere (maintained throughout the reaction) to ca. 70°C. catalyst (UC1) (400 μl) added and diisocyanate (IC1) (152.4 g; 1.02 mol) added through the addition port as described in Example 1, and the mixture heated at a rate of 50°C per hour to a temperature of approximately 180°C. Catalyst (UC2) (stannous octoate) (0.8 g) was then added, the pressure reduced to 10 mbar and the mixture held at 180°C until the hydroxyl value fell to 10 mg(KOH).$g^{-1}$ (or less). The reaction mixture was allowed to cool to 140-150°C, the vacuum released and the product discharged and allowed to cool to ambient temperature to yield the product oligomer as a white, opaque solid.

Example 5 - SE4 : Preparation of an ester-terminated polyurethane structuring polymer by "post-esterification" route

This example includes the use of a higher functional isocyanate as component of the base polymer.

Dimer diol (DD1) (607.3 g; 1.11 mol) and methyl isostearate (Est1) (261.4 g; 0.85 mol) were charged to a reactor as described in Example 1 and the mixture was heated under an inert nitrogen atmosphere (maintained throughout the reaction) to ca. 70°C and catalyst (UC1) (400 μl) was then added, followed by the triisocyanate (IC2) (17.66g; 0.03 mol). Diisocyanate (IC1) (172.4 g; 1.02 mol) was added as described in Example 1 and the mixture heated at a rate of 50°C per hour up to 225°C. Catalyst TBT (440 μl) was then added and the mixture held at 225°C until the hydroxyl value fell to 10 mg(KOH).$g^{-1}$ (or less). The reaction mixture was allowed to cool to 140-150°C under nitrogen sparge and the product discharged and allowed to cool to ambient temperature. The product oligomer obtained was a light yellow, translucent solid.

Example 6 - SE5 : Preparation of an ester-terminated structuring polymer by "post-esterification" route.

This example includes the use of a amine-alcohol as component of the base polymer.

Dimer diol (DD1) (530.1 g; 0.97 mol) methyl isostearate (Est1) (244.7 g; 0.8 mol) and monoethanol-amine (A1) (28.6 g; 0.47 mol) were charged to a reactor as described in Example 1 and heated to 70°C. Catalyst (UC1) (400 μl) was then added, diisocyanate (IC1) (196.6g; 1.17 mol) added as described in Example 1, and the mixture heated at a rate of 50°C per hour up to 225°C, TBT (440 μl) was added and the mixture held at 225°C until the hydroxyl value fell to 10 mg(KOH).$g^{-1}$ (or less). The reaction mixture was allowed to cool to 140-150°C under nitrogen sparge and the product discharged and allowed to cool to ambient temperature as a light yellow, opaque solid.

Example 7 - SE6 : Preparation of an amide-terminated polyurea structuring polymer

In this example, amidation of the terminal amine groups is performed post - polymerisation; the fatty methyl ester serves initially as a solvent and diluent, becoming the source of terminal alkyl chains in the subsequent amidation step.

Dimer diamine (DD4) (627 g; 1.15 mol) and methyl isostearate (Est1) (242.8 g; 0.79 mol) were charged to a

reactor as described in Example 1 a and heated to 70°C. Catalyst (UC1) (400 μl) was then added, diisocyanate (IC1) (130.1 g; 0.77 mol) added as described in Example 1, and the mixture was heated at a rate of 60°C per hour to 225°C, TBT (440 μl) was added and the mixture held at 225°C until the amine value fell to 10 mg(KOH).g$^{-1}$ (or less). The reaction mixture was allowed to cool to 170 to 180°C under nitrogen sparge and the product discharged and allowed to cool to ambient temperature as a light brown, opaque solid.

Example 8 - SE20 : Preparation of a urethane-terminated polyurethane structuring polymer

This example includes the use of an oligomeric polyester polyol derived entirely from dimer acid, as component of the base polymer.

Dimer diol (DD1) (152.94 g; 0.56 mol), polyester polyol DD5 (509.79 g; 0.25 mol), 1,6 hexanediol (D1) (30.12 g; 0.25 mol) and isostearyl alcohol (Alc2) (137.64 g; 0.51 mol) were charged to a reactor as described in Example 1 and heated to 70°C. Catalyst (UC1) (400 μl) was then added followed by diisocyanate (IC1) (169.51 g; 1.01 mol) added through the addition port as described in Example 1 (during isocyanate dosing the viscosity increases and therefore it can be desired to heat the mixture to keep it liquid enough in case the exothermic energy is not enough to keep the mixture liquid). The mixture was heated at 50°C per hour up to 140°C and held for 2-3 hours after which the hydroxyl value was <7 mg(KOH).g$^{-1}$. The product was discharged and allowed to cool to ambient temperature to yield the oligomer as a light yellow rubbery translucent solid.

The cooled polymer products may be ground e.g. in a cryogenic centrifugal mill, to produce a powder form for ease of handling and subsequent incorporation into formulations.

Further polymeric gellants were made generally as described above using the materials and molar proportions (ratios) set out in Table 1 below.

Table 1 Compositions of structuring polymers SE1 - SE33

| SE No | Reagents | | | | | | | |
|---|---|---|---|---|---|---|---|---|
| | dimer/trimer | | diol(s)/amine(s) | | isocyanate(s) | | end group | |
| | type | amt | type | amt | type | amt | type | amt |
| SE1 | DD1<br>DD7 | 1<br>0.08 | D2<br>D7 | 0.44<br>0.11 | IC1 | 1.02 | Est2<br>Est5 | 0.41<br>0.41 |
| SE 2 | DD1 | 1 | - | - | IC1 | 1.33 | Alc2 | 0.67 |
| SE 3 | DD2 | 1 | - | - | IC1 | 1.2 | Alc1<br>Est2 | 1.9<br>0.55 |
| SE 4 | DD1 | 3.3 | - | - | IC1<br>IC2 | 2.1<br>0.1 | Est1 | 2.1 |
| SE 5 | DD1 | 1 | A1 | 0.5 | IC1 | 1.25 | Est1 | 0.5 |
| SE6 | DD4 | 1 | - | - | IC1 | 0.75 | Est1 | 0.5 |
| SE7.1 | DD1 | 1 | - | - | IC1 | 0.75 | Est3 | 0.5 |
| SE7.2 | | | - | - | | | Est1 | 0.5 |
| SE7.3 | | | - | - | | | Est2 | 0.5 |
| SE7.4 | | | - | - | | | Est4 | 0.5 |
| SE8 | DD1<br>DD3 | 1<br>0.33 | - | - | IC1 | 1 | Est1 | 0.67 |
| SE9 | DD1 | 1 | D4 | 0.33 | IC3 | 1 | Est1 | 0.67 |
| SE10 | DD1 | 1 | D2 | 0.25 | IC1 | 1 | Est1 | 0.5 |
| SE11 | DD1 | 1 | - | - | IC1<br>IC4 | 0.4<br>0.33 | Est1 | 0.33 |
| SE12 | DD1 | 1 | D1 | 0.375 | IC1 | 1.125 | Est1 | 0.5 |

(continued)

| SE No | Reagents | | | | | | | |
|---|---|---|---|---|---|---|---|---|
| | dimer/trimer | | diol(s)/amine(s) | | isocyanate(s) | | end group | |
| | type | amt | type | amt | type | amt | type | amt |
| SE13 | DD1 | 1 | D1<br>D3 | 0.42<br>0.33 | IC1 | 1.125 | Est1 | 0.66 |
| SE14 | DD4 | 1 | A2 | 0.14 | IC1 | 1.43 | Est1 | 0.57 |
| SE15 | DD1 | 1 | - | - | IC1 | 1.33 | Alc1 | 0.67 |
| SE16 | DD1 | 1 | - | - | IC1 | 0.75 | Alc3 | 0.5 |
| SE17 | DD1 | 1 | D1 | 0.33 | IC1 | 1 | Alc2 | 0.67 |
| SE18 | DD1 | 1 | - | - | IC1 | 1.33 | Alc4 | 0.67 |
| SE19 | DD1 | 1 | D3 | 0.33 | IC1 | 1.67 | Alc2 | 0.67 |
| SE20 | DD1<br>DD5 | 1<br>1 | D1 | 1 | IC1 | 4 | Alc2 | 2 |
| SE21 | DD1 | 1 | D1 | 0.14 | IC1 | 1.43 | Alc5 | 0.57 |
| SE22 | DD1 | 1 | D5 | 0.2 | IC1 | 1.6 | Alc5 | 1 |
| SE23 | DD1 | 1 | - | - | IC1 | 0.75 | IC5 | 0.5 |
| SE24 | DD1 | 1 | D2 | 0.5 | IC1 | 1.25 | Est1 | 0.5 |
| SE25 | DD1 | 1 | D1 | 0.5 | IC1 | 1.25 | Est1 | 0.5 |
| SE26 | DD1 | 1 | D1 | 0.33 | IC1 | 1 | Est1 | 0.67 |
| SE27 | DD1 | 1 | - | - | IC1 | 0.67 | Est1 | 0.67 |
| SE28 | DD1 | 1 | D2<br>D6 | 0.2<br>0.13 | IC1 | 1 | Est1 | 0.75 |
| SE29 | DD1<br>DD6 | 1<br>0.1 | - | - | IC1 | 0.72 | Est1 | 0.32 |
| SE30 | DD1 | 1 | D1 | 0.25 | IC1 | 1 | Est1 | 0.5 |
| SE31 | DD4 | 1 | | | IC1 | 1.33 | Ami1 | 0.67 |
| SE32 | DD1 | 1 | D2 | 0.5 | ICI | 1.63 | Alc2 | 1.1 |
| SE33 | DD1<br>DD8 | 1<br>0.75 | D2 | 0.5 | IC1 | 2.75 | Alc2 | 1.1 |

Example 9 - Preparation of a gelled oil/fragrance

Table 2 indicates the composition of some fragrance oils gelled with polymers selected from (Table 1) above.

Table 2 Example compositions of structured fragrance oils

| Ex No | Ingredient | % | Oligomer (ester route) | % | Appearance at RT | Appearance at 60°C |
|---|---|---|---|---|---|---|
| **E1** | Frg1 | 50 | SE21 | 50 | Clear, strong gel | Clear, strong gel |
| **E2** | Frg1 | 70 | SE12 (post) | 30 | Clear, strong gel | Clear, strong gel |
| **E3** | Frg1 | 80 | SE17 | 20 | Clear, strong gel | Clear, strong gel |
| **E4** | Frg2 | 60 | SE26 (pre) | 40 | Clear, strong gel | Clear, strong gel |
| **E5** | Frg2 | 80 | SE17 | 20 | Clear, strong gel | Clear, strong gel |
| **E6** | Frg3 | 80 | SE26 (pre) | 20 | Clear, strong gel | Clear, strong gel |

(continued)

| Ex No | Ingredient | % | Oligomer (ester route) | % | Appearance at RT | Appearance at 60°C |
|---|---|---|---|---|---|---|
| **E7** | Frg3 | 80 | SE17 | 20 | Clear, strong gel | Clear, strong gel |
| **E8** | Frg2 | 80 | SE33 | 20 | Clear, strong gel | Clear, strong gel |
| **E9** | Frg3 | 80 | SE33 | 20 | Clear, strong gel | Clear, strong gel |
| **E10** | Oil1 | 60 | SE7.2 (pre) | 40 | Clear strong gel | Clear strong gel |
| **E11** | Oil1 / Frg1 | 45/5 | SE26 | 50 | Clear strong gel | Clear strong gel |

The use of the suffix '(pre)' in Table 2 indicates the specific use of a "pre-esterification" route for synthesis of the structuring polymer
The use of the suffix '(post)' in Table 2 indicates the specific use of a "post-esterification" route for synthesis of the structuring polymer

Example 10 - Air Freshener: Hot plate method

The structuring polymer SE33 (2 g) was added to 8 grams of fragrance Frg2. The mixture was heated to 90°C by a hot plate, under stirring, until the polymer was completely dissolved. The mixture was then allowed to cool down whereupon a gel forms. To prevent loss of volatiles from the fragrance structuring polymer mix an air cooler or a reflux column was used. A clear strong gel without syneresis was formed that was stable up to 60°C for 2 weeks and showed no creep.

Example 11 - Fragranced gel candle: Hot plate method

Fragrance Frg1 (0.5 g) and 5 gram structuring polymer SE26 was added to 4.5 grams of Oil1. The mixture was heated up to 90°C under stirring and reflux until the polymer was completely dissolved. Once the structuring polymer was dissolved the mixture is allowed to cool down, whereupon it forms a gel structure. A clear strong gel was obtained that can be burned, releasing fragrance.

Example 12 - Air freshener: Microwave method

The structuring polymer SE21 (5 g) was added to 5 grams of fragrance Frg1 in a Petri-dish. The mixture was put into the microwave (Moulinex; type Y62) at 600-650 Watt for 1 minute. The mixture was taken out and homogenised (as only part of the polymer is dissolved) and put into the microwave again for 1 minute at 600-650 Watt. A clear strong gel with no syneresis was obtained, which was stable to 60°C for 2 weeks without showing creep. Microwave heating can also, if necessary, be followed by stirring and/or additional heating on a hot plate.

Example 13 - Preparation of structuring polymer/solvent mixtures

Fragrance oils typically contain solvent. This can be e.g. benzyl alcohol (CoS2), dipropylene glycol (CoS4), Dowanol TPM (CoS1)(tripropylene glycol mono methyl ether), Carbitol (CoS3)(diethylene glycol mono ethyl ether), Isopar M (CoS6), Isopar V (CoS5) and isopropylmyristate (CoS7). The exact nature of this solvent can have a marked effect on the performance of the structuring polymer, and is a factor in the selection of the polymer type.

The use of a co-solvent to reduce viscosity and melt point of the structuring polymer places an upper limit on the concentration of fragrance in the end product. This can be solved by using a more concentrated version of fragrance to compensate for the additional co-solvent in the system due to the solvent/gellant mixture. In some cases it may be possible to use the same co-solvent as that normally present in the fragrance oil : the temperature to which the polymer/solvent mixture has to be heated depends on the type and concentration of the solvent. Table 3 provides some examples of polymer/solvent mixtures effective in reducing the softening point of the structuring polymer:

Table 3 Structuring polymer/solvent mixtures

| Mixture | Oligomer | % | Solvent | % |
|---|---|---:|---|---:|
| M1 | SE17 | 50 | CoS2 | 50 |
| M2 | SE17 | 75 | CoS2 | 25 |

(continued)

| Mixture | Oligomer | % | Solvent | % |
|---|---|---|---|---|
| M3 | SE17 | 75 | CoS7 | 25 |
| M4 | SE26 | 50 | CoS7 | 25 |
| M5 | SE26 | 75 | CoS3 | 25 |

4 grams of structuring polymer SE17 was added to 4 grams of solvent CoS2. The mixture was heated to 90°C by a hot plate under stirring until the polymer SE17 was completely dissolved. The mixture was stored at 90°C.

Example 14 - Preparation of structured air freshener by'semi-cold' method

4 grams of M1 of 90°C was added to concentrated fragrance of type frg1 (6g) at room temperature and mixed under extensive stirring and reflux. The mixture was allowed to cool down. A clear strong gel without syneresis was formed that has temperature stability up to 60°C and no creep.

Example 15 - Preparation of structured air freshener by 'cold' method

In some instances, depending on the formulation of the fragrance/oil (including the choice of solvent), it is possible to incorporate the structuring polymer at lower temperatures than described in the previous examples, and even at ambient conditions. This has the advantage of reducing the loss of volatile components from the formulation without the need for reflux, and saves energy and preparation time. It also allows the incorporation of heat-sensitive ingredients into the fragrance recipe.

The M3 mixture in Table 3 was allowed to cool down from 100°C to room temperature. After some time the mixture will form a gel, but initially the mixture remains liquid at room temperature. The liquid M3 mixture (2.67 gram) at room temperature was added to the concentrated version of fragrance frg3 (7.33 gram) at room temperature under extensive stirring. The air freshener is allowed to rest, resulting in the formation of clear strong gel, with no syneresis, temperature stability up to 60°C and no creep. The advantage of this method is that no volatiles are lost from the fragrance.

Example 16 - In-situ polymerisation preparation method

In-situ polymerisation describes the preparation of the gellant polymer *within* the fragrance/solvent/oil matrix, so that a separate step to incorporate the gellant polymer is not required. Potential advantages include process simplicity and cost savings, also use of more cross-linked systems to improve the gel properties. It is only possible when the fragrance/solvent/oil contains substantially compounds unreactive to the components of the polymerisation process; this includes esters, ethers, aldehydes, ketones and hydrocarbon-based compounds.

The reactants are dimerdiol, a modifying polyol and an end-capping moiety such as alcohol, an este-alcohol such as Alc5, or the product of esterification between a fatty acid and a molar excess of dimer diol. Three different processing routes have been tested, using polymer/solvent ratios of 50/50 to 80/20.

All components except the diisocyanate are combined. The diisocyanate is dosed and allowed to react.

Example 17 - Air freshener

2.23 grams DD1, 1.21 grams Alc2 and 0.18 gram D2 were added to 5 grams of the inert fragrance Frg2 in a Petri-dish. The catalyst UC1 (8 μl) was added and the mixture was homogenised. IC1 (1.37 grams) was dosed and the mixture was homogenised. The air freshener system was allowed to react at 40°C in an oven for 24 hours. After 24 hours a clear gel was formed which reached a temperature stability of 60°C and showed no creep.

Example 18 - Fragranced gel candle

4.47 grams DD1, 2.43 grams Alc2, 0.36 gram D2, 2 grams of Frg3 were added to 8 grams Oil1 in a 50 ml beaker. The catalyst UC1 (8 μl) was added and the mixture was homogenised. IC1 (2.74 gram) was dosed and the mixture was homogenised. The fragranced gel candle mixture was allowed to react at 60°C in an oven for 24 hours. After 24 hours a clear gel was formed when cooled to room temperature, which reached a temperature stability of 70°C.

In the following Examples, the ingredients and ratios are chosen so that the gellant polymer exhibits extensive cross-linking, which strengthens the gel structure but loses or reduces the thermo-reversible character

Example 19 - Alcohol cross-linked air-freshener

6.15 grams DD1, 0.20 gram D2, 0.61 gram D7 and catalyst UC1 (16 μl) were added to 10 grams of fragrance Frg3 in a Petri-dish. The mixture was homogenised, followed by addition of 3.04 grams IC1. The mixture was homogenised and allowed to react at 40°C for 24 hours. A slightly hazy strong gel was obtained which was stable up to 60°C without showing creep.

Example 20 - Isocyanate cross-linked air freshener

5.94 grams DD1, 0.20 gram D2 and catalyst UC1 (16 μl) were added to 23.34 grams of fragrance Frg3 in a Petri-dish. The mixture was homogenised, followed by addition of 1.47 grams IC1 and 2.39 grams IC2. The mixture was homogenised and allowed to react at 40°C for 24 hours. A clear strong gel was obtained which was stable up to 60°C without showing creep.

A hydroxyl-, isocyanate- or amine-ended pre-polymer/oligomer is prepared according to the methods described above. This pre-polymer is added to the fragrance, optionally with additional monomers and end-cappers/chain stoppers.

Example 21 - Hydroxyl terminated Polymeric fatty acid dimer/trimer moiety for production of a structuring polymer for a gel candle

The fatty acid dimer/trimer moiety used is of type SE26, but without the addition of an end-capping moiety. 9.32 grams of molten SE26 (T = 90°C), without end-cap, was added to 15 grams of pre-heated (90°C) paraffin. The catalyst UC1 (20 μl) was added and the mixture was homogenised and IC1 (0.68 gram) was added and the mixture was homogenised again. The mixture was allowed to react at 80°C for 24 hours. A transparent strong gel was obtained when cooled down to room temperature.

Example 22 - Amine terminated polymeric fatty acid dimer/trimer moiety for production of a structuring polymer for an air freshener

The fatty acid dimer/trimer moiety used is of type SE6, but without the addition of an end-capper. 14.1 grams of molten SE6 (temperature is 70°C), without end cap, was added to 15 grams of fragrance Frg2 at 60°C, followed by addition of catalyst SO (24 μl). The isocyanate IC1 (0.9 grams) was dosed and the mixture was homogenised. The mixture was allowed to react at 60°C in an oven. A slightly hazy gel was obtained, which showed no creep at 50°C.

Example 23 - Fragrance release curves for air fresheners made from Polymer SE32

Concentrated gels were made from three different fragrances (Frg1, Frg2 and Frg3) in polymer type SE32, using the preparation method outlined in Example 9 above. In all three cases, the weight ratio of the fragrance to the polymer was 4:1, i.e. 8g of fragrance to 2g of polymer. A reflux condenser was used during preparation to ensure no loss of volatile fragrance material from the reaction mixture.

The resultant molten air freshener gels were poured in to glass dishes of a 40mm diameter, such that each dish contained 3.63g ± 0.02g. The gel was cooled quickly to ambient temperature, and duplicate samples placed in a climate room set at a temperature of 22.5°C ± 0.5°C and a relative humidity of 50% ± 5%. The weights of the glass dishes plus gel sample were measured daily, using a Mettler AT200 digital scale. The corresponding neat fragrance oils were tested in parallel to act as an experimental control. Fragrance release curves for the three gel formulations are shown in Figures 1, 2 and 3.

These figures illustrate the ability of the single polymer type to structure fragrances of different polarities. It can be seen that the rate of fragrance release in the gel is lower than for the neat fragrance oil, this can clearly be attributed to the structuring action of the polymer, while remaining relatively constant over the test period of 18 days; the effective lifetime of the fragrance- releasing article is thereby considerably extended.

## Claims

**1.** A structured composition comprising an organic phase comprising a fragrance composition,

- the organic phase being structured by a structuring polymer,
- wherein the structuring polymer contains fatty acid dimer/trimer containing moieties and comprises urea and/or urethane linkages, and wherein the structuring polymer has a molecular mass of from 1,500 to 10,000 Da.

2. The structured composition of claim 1 wherein said fatty acid dimer/trimer containing moieties are polyol derivatives of fatty acid dimers and/or trimers, polyamine derivatives of fatty acid dimers and/or trimers, and/or polymeric moieties which include dimer/trimer acid derivatives.

3. The structured composition of claim 1 or 2 wherein the structuring polymer is at least partially branched or cross-linked.

4. The structured composition of any preceding claim wherein the structuring polymer comprises from 2 to 100 urethane or urea linkages.

5. The structured composition of any preceding claim wherein the structuring polymer comprises from 2 to 50 fatty acid dimer/trimer containing moieties.

6. The structured composition of any preceding claim wherein the structuring polymer comprises the following constituents in the relative quantities indicated in molar equivalents where;

1)

$$DI = N$$

2)

$$(N-1) \leq (D+d) \leq (N+1)$$

3)

$$EC1 = (DI - D - d + 1)$$

4)

$$d < D$$

where,
N = from 2 to 10 mole equivalents
DI = mole equivalents diisocyanate
D = mole equivalents of fatty acid dimer/trimer containing moiety d = mole equivalents polymer modifying moiety
EC1 = mole equivalent of a capping moiety which reacts, or is reacted, only with the diisocyanate moiety.

7. The structured composition of any one of claims 1 to 5 wherein the structuring polymer comprising the following constituents in the relative quantities indicated in molar equivalents;

1)

$$DI = N$$

2)

$$(D+d) = N+1$$

3)

$$0 \leq EC2 \leq 2$$

4)

$$d < D$$

where,
N = from 2 to 10 mole equivalents
DI = mole equivalents diisocyanate
D = mole equivalents fatty acid dimer/trimer containing moiety d = mole equivalents polymer modifying moiety
EC2 = mole equivalents of a capping moiety which reacts, or is reacted, only with the dimer/trimer containing moiety and the polymer modifying moiety if present.

**8.** The structured composition of any preceding claim which is generally clear or transparent.

**9.** A method of forming a structured composition comprising an organic phase comprising a fragrance composition, the method comprising the steps of:

- providing a structuring polymer which contains fatty acid dimer/trimer containing moieties and which comprises urea and/or urethane linkages, wherein the structuring polymer has a molecular mass of from 1,500 to 10,000 Da;
- providing an organic phase comprising a fragrance composition; and
- combining the structuring polymer with the organic phase under suitable conditions to form a structured composition.

**10.** The method of claim 9 wherein the structuring polymer is combined with the organic phase comprising a fragrance composition at a temperature of from ambient temperature to 140 °C.

**11.** Use of a structuring polymer which contains fatty acid dimer/trimer containing moieties and comprises urea and/or urethane linkages, in the manufacture of a structured composition comprising an organic phase which comprises a fragrance composition, wherein the structuring polymer has a molecular mass of from 1,500 to 10,000 Da.

**Patentansprüche**

**1.** Strukturierte Zusammensetzung, umfassend eine organische Phase mit einer Duftstoffzusammensetzung,

- wobei die organische Phase mit einem strukturierenden Polymer strukturiert wird,
- wobei das strukturierende Polymer fettsäuredimer- bzw. -trimerhaltige Gruppen enthält und Harnstoff - und/oder Urethanverkettungen umfasst, und wobei das strukturierende Polymer eine Molekülmasse von 1.500 bis 10.000 Da aufweist.

**2.** Strukturierte Zusammensetzung nach Anspruch 1, wobei die fettsäuredimer- bzw. -trimerhaltigen Gruppen Polyolderivate von Fettsäuredimeren und/oder -trimeren, Polyaminderivate von Fettsäuredimeren und/oder -trimeren und/oder Polymergruppen sind, die Dimer- bzw. Trimersäurederivate einschließen.

**3.** Strukturierte Zusammensetzung nach einem der Ansprüche 1 oder 2, wobei das strukturierende Polymer mindestens teilweise verzweigt oder vernetzt ist.

**4.** Strukturierte Zusammensetzung nach einem der vorhergehenden Ansprüche, wobei das strukturierende Polymer 2 bis 100 Urethan- oder Harnstoffverkettungen umfasst.

**5.** Strukturierte Zusammensetzung nach einem der vorhergehenden Ansprüche, wobei das strukturierende Polymer 2 bis 50 fettsäuredimer- bzw. -trimerhaltige Gruppen umfasst.

**6.** Strukturierte Zusammensetzung nach einem der vorhergehenden Ansprüche, wobei das strukturierende Polymer

die folgenden Bestandteile in den relativen Mengen, die angegeben sind als molare Äquivalente, umfasst, worin

1)

$$DI = N$$

2)

$$(N-1) \leq (D+d) \leq (N+1)$$

3)

$$EC1 = (DI-D-d+1)$$

4)

$$d<D$$

worin
N = 2 bis 10 Moläquivalente ist
DI = moläquivalentes Diisocyanat ist,
D = Moläquivalente der fettsäuredimer- bzw. -trimerhaltigen Gruppe ist,
d = Moläquivalente der polymermodifizierenden Gruppe ist,
EC1 = das Moläquivalent einer verkapselten Gruppe ist, die nur mit der Diisocyanat-Gruppe reagiert oder reagiert wird.

**7.** Strukturierte Zusammensetzung nach einem der Ansprüche 1 bis 5, wobei das strukturierende Polymer die folgenden Bestandteile in den relativen Mengen, die angegeben sind als molare Äquivalente, umfasst;

1)

$$DI = N$$

2)

$$(D+d) = N+1$$

3)

$$0 \leq EC2 \leq 2$$

4)

$$d<D$$

worin
N = 2 bis 10 Moläquivalente ist DI = moläquivalentes Diisocyanat ist,
D = moläquivalente fettsäuredimer- bzw. -trimerhaltige Gruppe ist,

d = Moläquivalente der polymermodifizierenden Gruppe ist,
EC2 = Moläquivalente einer verkapselten Gruppe ist, die nur mit der dimer- bzw. trimerhaltigen Gruppe und der polymermodifizierenden Gruppe, wenn vorhanden, reagiert oder reagiert wird.

**8.** Strukturierte Zusammensetzung nach einem der vorhergehenden Ansprüche, die im Allgemeinen klar oder transparent ist.

**9.** Verfahren zum Bilden einer strukturierten Zusammensetzung, umfassend eine organische Phase mit einer Duftstoffzusammensetzung, wobei das Verfahren die folgenden Schritte umfasst:

- Bereitstellen eines strukturierenden Polymers, das fettsäuredimer- bzw. -trimerhaltige Gruppen enthält und Harnstoff- und/oder Urethanverkettungen umfasst, wobei das strukturierende Polymer eine Molekülmasse von 1.500 bis 10.000 Da aufweist;
- Bereitstellen einer organischen Phase, umfassend eine Duftstoffzusammensetzung; und
- Verbinden des strukturierenden Polymers mit der organischen Phase unter geeigneten Bedingungen, um eine strukturierte Zusammensetzung zu bilden.

**10.** Verfahren nach Anspruch 9, wobei das strukturierende Polymer mit der organischen Phase, umfassend eine Duftstoffzusammensetzung, bei einer Temperatur von Umgebungstemperatur bis 140 °C verbunden wird.

**11.** Verwendung eines strukturierenden Polymers, das fettsäuredimer- bzw. -trimerhaltige Gruppen enthält und Harnstoff- und/oder Urethanverkettungen umfasst, für die Herstellung einer strukturierten Zusammensetzung, umfassend eine organische Phase mit einer Duftstoffzusammensetzung, wobei das strukturierende Polymer eine Molekülmasse von 1.500 bis 10.000 Da aufweist.

**Revendications**

**1.** Composition structurée comportant une phase organique comprenant une composition de parfum,

- la phase organique étant structurée par un polymère structurant,
- dans laquelle le polymère structurant contient des fractions contenant des dimères/trimères d'acides gras et comprend des liaisons urée et/ou uréthane et dans laquelle le polymère structurant a une masse moléculaire de 1 500 à 10 000 Da.

**2.** Composition structurée selon la revendication 1 dans laquelle lesdites fractions contenant des dimères/trimères d'acides gras sont des dérivés polyols de dimères et/ou trimères d'acides gras, des dérivés polyamines de dimères et/ou trimères d'acides gras et/ou des fractions polymères qui comprennent des dérivés d'acides dimères/trimères.

**3.** Composition structurée selon la revendication 1 ou 2 dans laquelle le polymère structurant est au moins partiellement ramifié ou réticulé.

**4.** Composition structurée selon l'une quelconque des revendications précédentes dans laquelle le polymère structurant comprend de 2 à 100 liaisons uréthane ou urée.

**5.** Composition structurée selon l'une quelconque des revendications précédentes dans laquelle le polymère structurant comprend de 2 à 50 fractions contenant des dimères/trimères d'acides gras.

**6.** Composition structurée selon l'une quelconque des revendications précédentes dans laquelle le polymère structurant comprend les constituants suivants dans les quantités relatives indiquées en équivalents molaire :

1)

$$DI = N$$

2)

$$(N-1) \leq (D + d) \leq (N+1)$$

3)

$$EC1 = (DI - D - d + 1)$$

4)

$$d < D$$

où
N = de 2 à 10 équivalents en mole,
DI = équivalents de diisocyanate en mole,
D = équivalents de fraction contenant des dimères/trimères d'acides gras en mole,
d = équivalents de fraction modifiant le polymère en mole,
EC1 = équivalent en mole d'une fraction coiffante qui réagit, ou est amenée à réagir, seulement avec la fraction diisocyanate.

**7.** Composition structurée selon l'une quelconque des revendications 1 à 5 dans laquelle le polymère structurant comprend les constituants suivants dans les quantités relatives indiquées en équivalents molaire :

1)

$$DI = N$$

2)

$$(D + d) = N + 1$$

3)

$$0 \leq EC2 \leq 2$$

4)

$$d < D$$

où
N = de 2 à 10 équivalents en mole,
DI = équivalents de diisocyanate en mole,
D = équivalents de fraction contenant des dimères/trimères d'acides gras en mole,
d = équivalents de fraction modifiant le polymère en mole,
EC2 = équivalents en mole d'une fraction coiffante qui réagit, ou est amenée à réagir, seulement avec la fraction contenant des dimères/trimères et la fraction modifiant le polymère si elle est présente.

**8.** Composition structurée selon l'une quelconque des revendications précédentes qui est généralement limpide ou transparente.

**9.** Procédé de formation d'une composition structurée comprenant une phase organique comprenant une composition

de parfum, le procédé comprenant les étapes consistant à :

- obtenir un polymère structurant qui contient des fractions contenant des dimères/trimères d'acides gras et qui comprend des liaisons urée et/ou uréthane, le polymère structurant ayant une masse moléculaire de 1 500 à 10 000 Da ;
- obtenir une phase organique comprenant une composition de parfum ; et
- combiner le polymère structurant avec la phase organique dans des conditions appropriées pour former une composition structurée.

**10.** Procédé selon la revendication 9 dans lequel le polymère structurant est combiné avec la phase organique comprenant une composition de parfum à une température allant de la température ambiante à 140 °C.

**11.** Utilisation d'un polymère structurant qui contient des fractions contenant des dimères/trimères d'acides gras et qui comprend des liaisons urée et/ou uréthane, dans la fabrication d'une composition structurée comprenant une phase organique qui comprend une composition de parfum, le polymère structurant ayant une masse moléculaire de 1 500 à 10 000 Da.

# Fig.1.

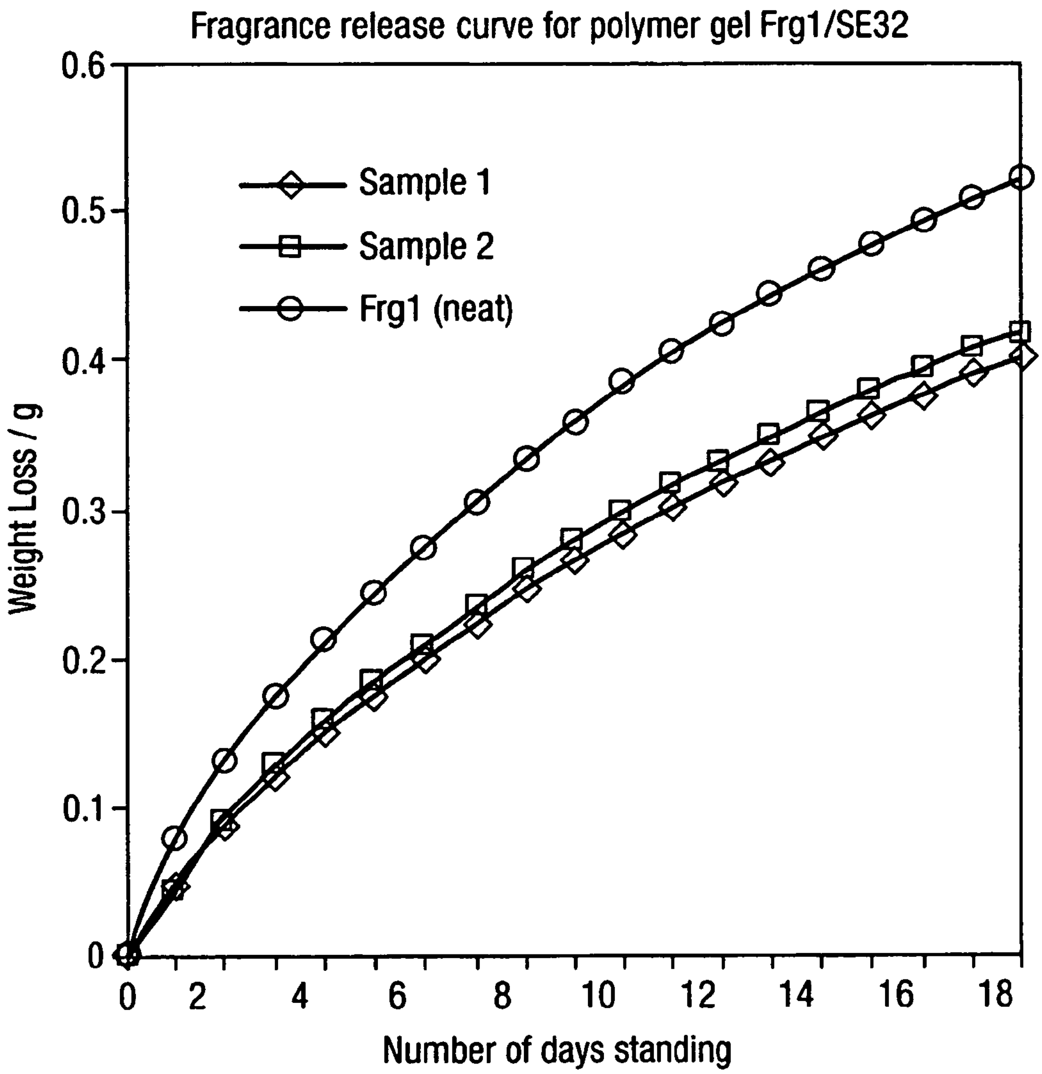
Fragrance release curve for polymer gel Frg1/SE32
Sample 1
Sample 2
Frg1 (neat)
Weight Loss / g
0.6
0.5
0.4
0.3
0.2
0.1
0
0
2
4
6
8
10
12
14
16
18
Number of days standing

Fig.2.

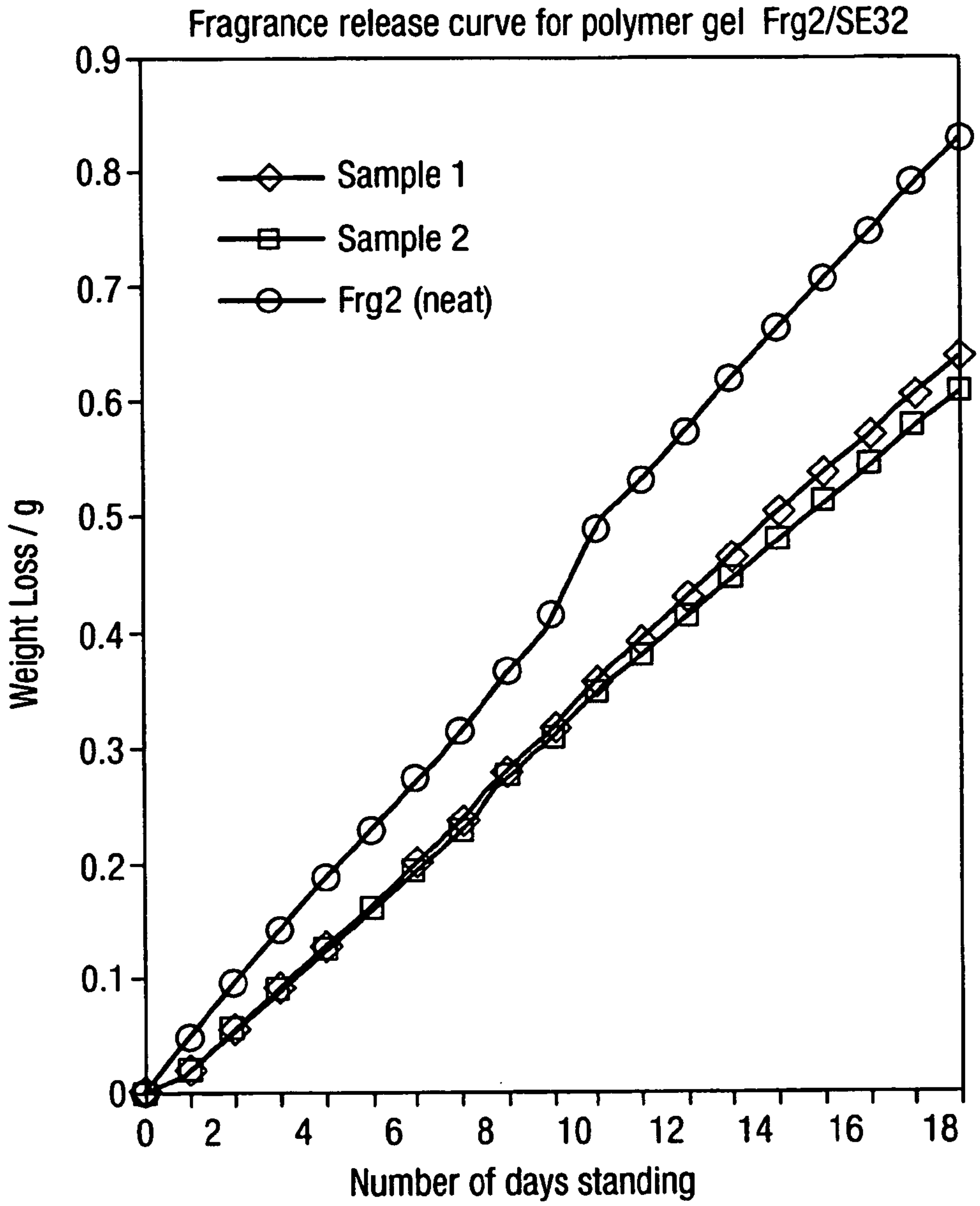
Fragrance release curve for polymer gel Frg2/SE32
Sample 1
Sample 2
Frg2 (neat)
Weight Loss / g
0
0.1
0.2
0.3
0.4
0.5
0.6
0.7
0.8
0.9
0
2
4
6
8
10
12
14
16
18
Number of days standing

# Fig.3.

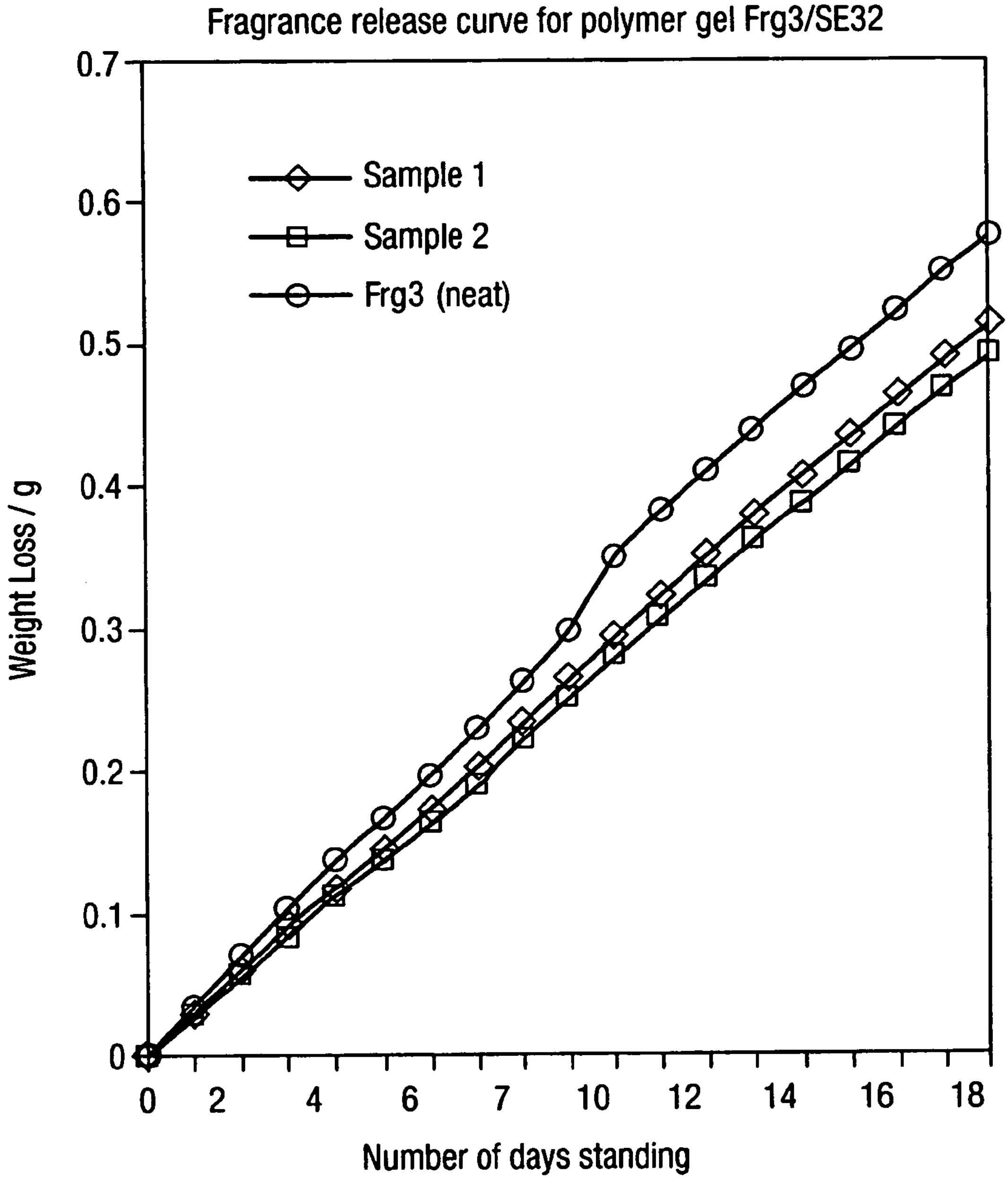
Fragrance release curve for polymer gel Frg3/SE32
Sample 1
Sample 2
Frg3 (neat)
Weight Loss / g
0
0.1
0.2
0.3
0.4
0.5
0.6
0.7
0
2
4
6
7
10
12
14
16
18
Number of days standing

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004081056 A